(19) Europäisches Patentamt
European Patent Office
Office européen des brevets

(11) **EP 3 682 910 B1**

(12) **EUROPEAN PATENT SPECIFICATION**

(45) Date of publication and mention
of the grant of the patent:
**01.09.2021 Bulletin 2021/35**

(51) Int Cl.:
***A61L 9/12*** *(2006.01)* ***B60H 3/00*** *(2006.01)*

(21) Application number: **19152227.5**

(22) Date of filing: **17.01.2019**

(54) **AIR FRESHENER WITH SCENT INTENSITY SELECTOR**

LUFTERFRISCHER MIT DUFTINTENSITÄTSWAHLSCHALTER

RAFRAÎCHISSEUR D'AIR DOTÉ D'UN SÉLECTEUR D'INTENSITÉ DE PARFUM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**22.07.2020 Bulletin 2020/30**

(73) Proprietor: **The Procter & Gamble Company
Cincinnati, OH 45202 (US)**

(72) Inventors:
• **GOVINDAN RADHAKRISHNAN, Kumaresh
138547 Singapore (SG)**
• **LOW, Bee Ting
138547 Singapore (SG)**
• **CHO, Mei San Gigi
138547 Singapore (SG)**

(74) Representative: **P&G Patent Belgium UK
N.V. Procter & Gamble Services Company S.A.
Temselaan 100
1853 Strombeek-Bever (BE)**

(56) References cited:
US-A- 4 666 638          US-A- 5 478 505
US-A1- 2010 308 126      US-A1- 2017 087 266

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to the field of air fresheners capable of varying scent intensity and particularly relates to an air freshener with a scent intensity selector, and a method of varying scent intensity.

BACKGROUND OF THE INVENTION

Air fresheners are commonly used to provide a pleasant aroma in an interior occupancy space. There are air fresheners which have features for adjusting fragrance intensity. For example, there are air fresheners which comprise a container containing a gel, a lid covering the container and a regulating plate between the gel and the lid wherein the lid can be rotated relative to the container between an open position and a closed position with respect to the regulating plate. The lid and the regulating plate has openings thereon. The openings of the regulating plate are aligned with the openings of the lid in an open position to dispense the volatile material through the aligned openings. The openings of the regulating plate are not aligned with the openings of the lid in the closed position to inhibit dispensing of the volatile material. However, due to imperfections in dimensions of components that make up the air fresheners, gaps may exist in the air fresheners resulting in unintended air flow diverted to the gaps which result in ineffective control of air flow through the aligned openings and ineffective control of the fragrance intensity. Documents US 5 478 505 A, US 2010/308126 A1 and US 4 666 638 A show examples of known air fresheners.
Therefore, there remains a need to provide an air freshener that provides effective scent control to vary fragrance intensity according to different user preferences while also providing a well-balanced scent experience that is customizable to individual users based on the user's preferences or in response to a malodor event in a non-energized condition, i.e. passive air flow in the environment which the air freshener is placed.

SUMMARY OF THE INVENTION

The present invention relates to an air freshener capable of varying scent intensity, the air freshener operably connectable to a mounting portion for releasably attaching the air freshener in an interior space, the air freshener comprising:

(a) a housing having a front housing part and a housing shroud, wherein the housing shroud circumscribes at least part of the front housing part to define a side of the housing;
(b) a center longitudinal axis extending along a length (L) of the front housing part and extending through a center of the front housing part;
(c) a diffusion window disposed in the housing shroud, the diffusion window having a diffusion window cross sectional plane extending across the diffusion window, wherein the diffusion window sectional plane has a diffusion window centroid;
(d) a perfume containing body contained within the housing in fluid communication with the diffusion window, wherein the perfume containing body comprises an exterior evaporative surface;
(e) an air flow axis intersecting the diffusion window centroid defining a diffusion path, wherein the defined diffusion path is at least partially formed by the exterior evaporative surface of the perfume containing body, wherein a diffusion path forming exterior evaporative surface is in fluid communication with the defined diffusion path and has a diffusion path forming exterior evaporative surface cross sectional area of 5 cm$^2$ to 100 cm$^2$;
(f) wherein the defined diffusion path comprises a minimum cross-sectional area of at least 0.2 cm$^2$ measured along the length of the air flow axis and at least along the length of the diffusion path forming exterior evaporative surface; and
(g) a scent intensity selector adjustably attached to the housing shroud for at least partially covering or uncovering the diffusion window.

BRIEF DESCRIPTION OF DRAWINGS

FIG. 1 is a front perspective view of an air freshener capable of varying scent intensity according to the present invention when a diffusion window of the air freshener is in a fully open position;
FIG. 2A is a side schematic view of the air freshener of FIG. 1;
FIG. 2B is a front schematic view of the air freshener of FIG. 1;
FIG. 3A is a front schematic view of a partial assembly of an air freshener according to the present invention;

FIG. 3B is a side schematic view of a first diffusion window of the air freshener of FIG. 3A;

FIG. 3C is a side schematic view of a second diffusion window of the air freshener of FIG. 3A;

FIG. 4A is a front perspective view of a variation of an air freshener according to the present invention;

FIG. 4B is a side schematic view of the air freshener of FIG. 4A;

FIG. 5 is a perspective view of components of a dispensing device for an air freshener according to the present invention;

FIG. 6A is front view of a partial assembly of an air freshener according to the present invention with the scent intensity selector in a first position (first vapor release state or "ON state") of the air freshener);

FIG. 6B is a side section view of the air freshener of FIG. 6A;

FIG. 7A is a front view of the air freshener of FIG. 6A with the scent intensity selector in a second position (second vapor release state of the air freshener);

FIG. 7B is a side section view of the air freshener of FIG.7A;

FIG. 8A is a front view of the air freshener of FIG. 6A with the scent intensity selector in a third position (third vapor release state of the air freshener);

FIG. 8B is a side section view of the air freshener of FIG.8A;

FIG. 9A is a front view of the air freshener of FIG. 6A with the scent intensity selector in a fourth position (fourth vapor release state of the air freshener);

FIG. 9B is a side section view of the air freshener of FIG.9A;

FIG. 10A is a front view of the air freshener of FIG. 6A with the scent intensity selector in a fifth position (fifth vapor release state of the air freshener);

FIG. 10B is a side section view of the air freshener of FIG.10A;

FIG. 11A is a side schematic view of an air freshener according to the present invention having a different scent intensity selector design;

FIG. 11B is a partial front schematic view of the air freshener of FIG. 11A;

FIG. 12 is a perspective view of components of an exemplary air freshener capable of varying scent intensity according to the present invention with the perfume containing body removed;

FIG. 13 is a perspective view of components of a perfume containing body for an air freshener according to the present invention;

FIG. 14 is a front perspective view of the air freshener of FIG. 3A in use in an automobile environment;

FIG. 15 is an exemplary air freshener according to the present invention;

FIG. 16 is a corresponding graph of Table 4 plotting cumulative evaporation of volatile materials released from inventive samples according to the air freshener of FIG. 15 with the diffusion window uncovered at different percentages as a function of time;

FIG. 17 is a graph plotting cumulative evaporation of volatile materials released from inventive samples according to the air freshener of FIG. 15 and inventive samples according to the air freshener having a dispensing device of FIG. 12 with the diffusion windows uncovered at different percentages as a function of time;

FIG. 18 is a corresponding graph of Table 7 plotting cumulative evaporation of volatile materials released from inventive samples according to the air freshener of FIG. 15 having different gaps as a function of the diffusion window uncovered at different percentages;

FIG. 19 is a corresponding graph of Table 8 plotting cumulative evaporation of volatile materials released from inventive samples according to the air freshener of FIG. 15 having different gaps as a function of different diffusion window cross-sectional areas; and

FIG. 20 is a corresponding graph of Table 6 plotting cumulative evaporation of volatile materials released from inventive samples according to the air freshener of FIG. 4 with the diffusion window uncovered at different percentages as a function of time.

## DETAILED DESCRIPTION OF THE INVENTION

The present invention relates to an air freshener and a method of varying scent intensity in an interior space in a user-friendly manner. The air freshener includes a housing having a scent intensity selector slidably coupled to the housing through a support located at a side of the housing. Specifically, the housing has a front housing part and the support is a housing shroud disposed around the front housing part to define a side of the housing. A diffusion window is disposed in the housing shroud, the diffusion window having a diffusion window cross sectional plane extending across the diffusion window, wherein the diffusion window sectional plane has a centroid. A perfume containing body is contained within the housing in fluid communication with the diffusion window, wherein the perfume containing body comprises an exterior evaporative surface. The scent intensity selector is adjustably attached to the housing shroud for at least partially covering or uncovering the diffusion window. Specifically, the user may adjust the scent intensity selector so that the diffusion window can be in a fully opened position, a fully closed position, a partially closed position or any position therebetween.

An air flow axis intersects the centroid of the diffusion window defining a diffusion path; wherein the defined diffusion path is at least partially formed by the exterior evaporative surface of the perfume containing body. The diffusion path forming exterior evaporative surface is in fluid communication with the defined diffusion path and has a diffusion path forming exterior evaporative surface cross sectional area of 5 cm$^2$ to 100 cm$^2$. The diffusion window cross sectional plane has a minimum cross-sectional area relative to the diffusion path forming exterior evaporative surface cross sectional area; wherein the defined diffusion path comprises a minimum cross-sectional area of at least 0.2 cm$^2$ measured along the length of the air flow axis and at least along the length of the diffusion path forming exterior evaporative surface.

A technical effect of the diffusion window, the defined diffusion path of a minimum cross-sectional area of at least 0.2 cm$^2$ measured along the length of the air flow axis and at least along the length of the diffusion path forming exterior evaporative surface, and the scent intensity selector is that the claimed combination of features achieves scent intensity variation and control in an air freshener. Variability of scent intensity in the air freshener enables an air freshening effect to be tailored to the preferences of the user.

In the following description, the air freshener described is a consumer product, such as an automobile air freshener, for evaporating a perfume composition in an interior occupancy space of an automobile to deliver a variety of benefits such as mold odor prevention, freshening, malodor removal or scenting of air in an interior occupancy space such as a vehicle passenger compartment space. However, it is contemplated that the air freshener may be configured for use in a variety of applications to deliver a volatile composition to provide the benefits in interior occupancy spaces such as rooms in household and commercial establishments with air conditioning systems, and the air freshener may include but is not limited to consumer products, such as, for example air freshening products, air fresheners or the like.

The air freshener may also comprise a delivery member configured to contain a liquid phase of the perfume composition and allow the liquid phase of the perfume composition to evaporate therefrom. The delivery member may include a wick, a membrane, gel, porous or semi-porous substrate including a felt pad. An exemplary delivery member may be a membrane which is a semi-permeable material which allows some components of matter to pass through but stops other components. Of the components that pass through, the membrane moderates the permeation of components i.e. some components permeate faster than other components. Such components may include molecules, ions or particles.

For the purposes of illustrating the present invention in detail, the invention is described below as a non-energized air freshener having a membrane in fluid communication with the volatile composition. However, it will be appreciated that the volatile composition may be delivered from the air freshener to the space through a wick. Further, the air freshener of the present invention can be energized or non-energized. Prior to describing the present invention in detail, the following terms are defined for clarity. Terms not defined should be given their ordinary meaning as understood by a skilled person in the relevant art.

"Interior space" as used herein, refers to a finite volume of space in a residential, commercial or automobile environment.

"Membrane" as used herein, refers to a semi-permeable material which allows some components of matter to pass through but stops other components. Of the components that pass through, the membrane moderates the permeation of components i.e. some components permeate faster than other components. Such components may include molecules, ions or particles.

"Microporous membrane" as used herein, refers to a material having a network of pores.

"Non-energized" means that the air freshener is passive and does not require to be powered by a source of external energy. In particular, the air freshener does not need to be powered by a source of heat, gas or electrical current. The air freshener may also be configured as an energized device. An exemplary energized device may be an electrical device. The energized device may be an electrical car outlet or battery-operated air freshener having a wick and/or a membrane as described in the following description to transport a volatile composition and/or evaporate a volatile composition therefrom; or other heating devices (e.g. devices powered by chemical reactions such as catalyst fuel systems; solar powered devices, etc.).

"Perfume containing body" as used herein, refers to a material that is vaporizable at room temperature and atmospheric pressure without the need of an additional energy source. The perfume containing body may be configured for various uses, including but not limited to, air freshening, deodorization, odor elimination, malodor counteraction, pest control, insect control, insect repelling, medicines/medicaments, disinfectants, sanitization, mood enhancement, aromatherapy aid, scented compositions, non-scented compositions, or any other use which requires a volatile composition that acts to condition, modify, or otherwise change the atmosphere or the environment. Further, it is not necessary for all the component materials of the volatile composition to be volatile. Any suitable volatile composition in any amount or form, including a liquid, solid, gel or emulsion, may be used. Materials suitable for use herein may include non-volatile compounds, such as carrier materials (e.g., water, solvents, etc.). It should also be understood that when the volatile composition is described herein as being "delivered", "emitted", or "released", this refers to the volatization of the volatile component thereof and does not require that the non-volatile components thereof be emitted.

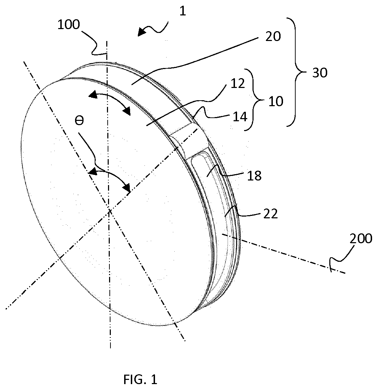

FIG. 1 is a front perspective view of an air freshener 1 according to the present invention. The air freshener 1 comprises a width, length and depth along an x-axis, y-axis and z-axis, respectively. The width, length and depth may be such that the air freshener 1 is considered compact and/portable. By "compact" or "portable", it is meant that the air freshener 1 is sized to be handheld. The air freshener 1 can be constructed as a disposable, single-use item or one that is refillable with a perfume containing body 2.

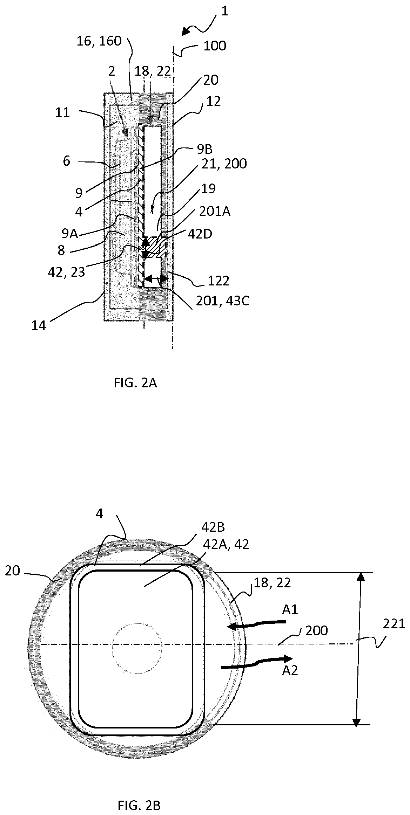

The air freshener 1 includes a housing 10 and a scent intensity selector 20 adjustably attached to the housing 10 to define a dispensing device 30 for delivering a volatile composition. The housing 10 is configured for containing a perfume containing body 2 comprising the volatile composition 8. An exemplary perfume containing body 2 may be in the form of a volatile composition cartridge described hereinafter with reference to FIG. 2A. The perfume containing body 2 may also comprise entirely of the volatile composition in a solid or semi-solid form.

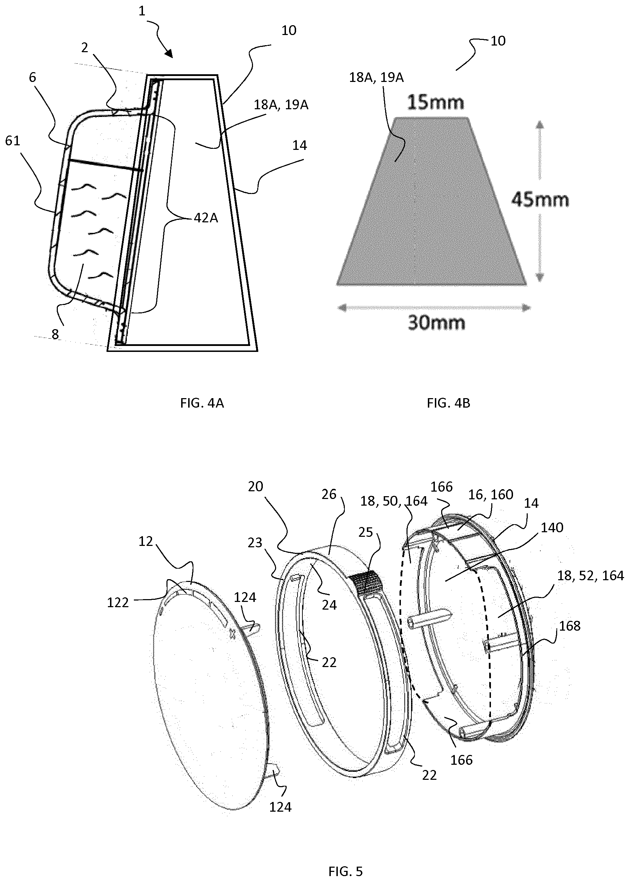

The housing 10 has a front housing part 12 defining a face of the housing 10 and a center longitudinal axis 100 of the front housing part 12 extends along the length of the front housing part 12 and extending through a center of the front housing part 12. The housing 10 may comprise a rear housing part 14 opposing the front housing part 12. The air freshener 1 has a diffusion window 18 for air flow through the housing 10 and the scent intensity selector 20 is slidably attached to a side of the housing 12 for at least partially covering or uncovering the diffusion window 18 to adjust an air flow through the housing 10. The scent intensity selector 20 may be adjustably attached to the side of the housing 10 by the scent intensity selector 20 having an annular shape corresponding to the side of the housing 12 (such as shown in FIG. 5) so as to be slidable around the side of the housing 10 between positions relative to the front housing part 12.

Referring to FIG. 1, the scent intensity selector 20 is adapted to have a range of adjustable positions relative to the housing 10, wherein a desired scent intensity setting is defined by the position of the scent intensity selector 20 along the range of adjustable positions. When the scent intensity selector 20 is rotated at an angle $\theta$ relative to the center longitudinal axis 100 and a center point of the front housing part 12 to a position such that the selector opening 22 is aligned with the diffusion window 18, the air freshener 1 is in a maximum intensity setting, i.e. there is maximum air flow through a housing interior 11 (see FIG. 2A) to the environment. FIG. 1 shows the air freshener 1 in a maximum scent intensity setting in which the diffusion window 18 of the air freshener 1 has a maximum opening, details of which is explained hereinafter with reference to FIG. 2A.

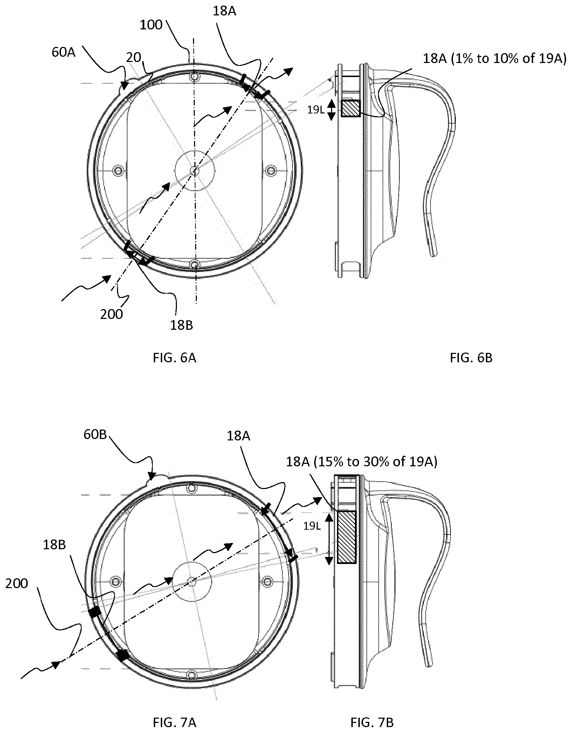

FIG. 2A is a side schematic view of the air freshener 1 of FIG. 1. The scent intensity selector 20 may comprise a selector opening 22 sized and shaped to correspond to the maximum opening of the diffusion window 18. An advantage is an opening of the diffusion window 18 can be adjusted by turning the scent intensity selector 20 and moving the selector opening 22 over at least a part of the diffusion window 18, details of which is further described with respect to FIGS. 6A to 10B. The side of the housing 10 is defined by a housing shroud 16 disposed about the front housing part 12. The housing shroud 16 has an inner wall 160 extending from the rear housing part 14 to define a housing interior 11 for receiving the at least a portion of the perfume containing body 2. The perfume containing body 2 comprises an exterior evaporative surface 4 arranged in fluid communication with the diffusion window 18. Specifically, the perfume containing body 2 may be a volatile composition refill comprising a container 6 containing a volatile composition 8. A membrane 9 comprising the exterior evaporative surface 4 covers the container 6 and is in fluid communication with the volatile composition 8. Accordingly, a first side 9A of the membrane 9 is in fluid communication with the volatile composition 8 and a second side 9B of the membrane 9 is the exterior evaporative surface 4.

Referring to FIG. 2A, the diffusion window 18 has a diffusion window cross sectional plane 19 extending across the diffusion window 18, wherein the diffusion window cross sectional plane 19 has a diffusion window centroid 21. Referring to FIG. 1 and FIG. 2A, an air flow axis 200 orthogonal to the center longitudinal axis 100 intersects the diffusion window centroid 21 defining a diffusion path 201. The defined diffusion path 201 is at least partially formed by the exterior evaporative surface 4 of the perfume containing body 2. The defined diffusion path 201 is in the housing interior while the diffusion window cross sectional plane 19 based on the diffusion window 18 which is on or at the housing exterior. A diffusion path forming exterior evaporative surface 42 is in fluid communication with the defined diffusion path 201. Further, the perfume containing body 2 may be arranged within the housing 10 such that the diffusion path forming exterior surface 42 defines a side of the diffusion window 18. The diffusion path forming exterior surface 42 may be aligned with an edge 23 of the selector opening 22 as shown in FIG. 2A. However, it will be appreciated that the diffusion path forming exterior evaporative surface 42 may be offset from the edge 43 but is in fluid communication with the defined diffusion path 201. The diffusion path forming exterior surface 42 may be the second side 9B of the membrane 9 when the air freshener 1 is activated and the volatile composition 8 wets the membrane 9 to form a wetted area of the membrane 9.

The selector opening 22 may comprises a length 221 (FIG. 2B) and width wherein the length of the selector opening may correspond to a length of the diffusion path forming exterior surface 42 and the width of the selector opening 22 may be defined by a gap 43C between the diffusion path forming exterior surface 42 and an inner surface 122 of the front housing part 12 that opposes the diffusion path forming exterior surface 42. The width of the selector opening 22 may be in the range of 4 mm to 11 mm, 5 mm to 10 mm, 6 mm to 9 mm, 7 mm or different combinations of the upper and lower numbers described before or combinations of any integer in the ranges listed above. The width of the diffusion window 18 may be the same as the width of the selector opening 22. The gap 43C may also correspond to the width of the selector opening 22.

The defined diffusion path 201 may comprise a minimum cross-sectional area 201A of at least 0.2 cm$^2$ measured along the length of the air flow axis 200 and at least along a length 42D of the diffusion path forming exterior evaporative surface 42. The minimum cross-sectional area 201A may be determined by multiplying the length 42D of the diffusion path forming exterior evaporative surface 42 and the gap 43C between the diffusion path forming exterior evaporative surface 42 and the inner surface 122 of the front housing part 12.

FIG. 2B is a front schematic view of the air freshener 1 of FIG. 1 without the front housing part 12. The diffusion path forming exterior evaporative surface 42 has a diffusion path forming exterior evaporative surface cross sectional area 42A of 5 cm$^2$ to 100 cm$^2$. It will be appreciated that the diffusion path forming exterior evaporative surface cross section area 42A varies according to a desired amount of volatile or perfume composition in the perfume containing body 2 and to suit different air freshener sizes. The volatile composition 8 may be a liquid perfume composition configured in an amount from 2 ml to 50 ml, from 4 ml to 30 ml, from 6 ml to 20 ml, from 6.5 ml to 8 ml or different combinations of the upper and lower numbers described before or combinations of any integer in the ranges listed above. The diffusion path forming exterior evaporative surface cross sectional area 42A may be measured as a portion of the membrane surface that is in fluid communication with the volatile composition 8. The membrane 9 may also include a non-wettable area 42B for sealing the membrane 9 to the container 6 of FIG. 2A.

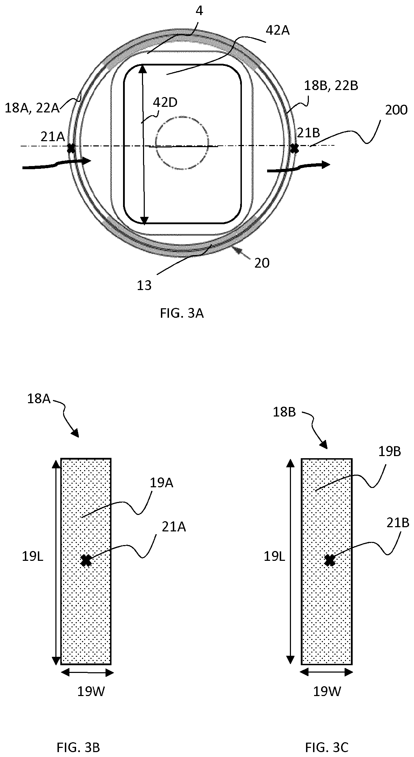

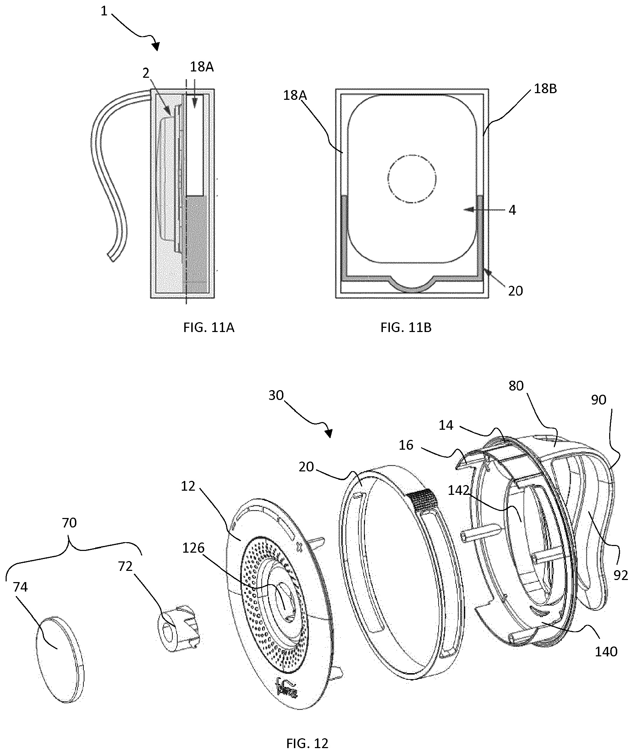

Arrows A1, A2 are shown in FIG. 2B as illustrations of exemplary directions of air flow entering and exiting along the air flow axis 200 when there is a single diffusion window 18 in the housing 10. The housing 10 may comprise a single diffusion window 18 in the housing shroud 16 as shown in FIGS. 1, 2A and 2B. The housing 10 may also comprise at least two diffusion windows 18 to promote air flow across diffusion path forming exterior evaporative surface 42 such as shown in FIGS. 3A, 3B, 3C. For the purposes of this disclosure, but without intending to limit the scope of the invention, the housing 10 is described as a structure having two diffusion windows 18, specifically first and second diffusion windows 18A, 18B. The diffusion windows 18A, 18B may be opposed (as shown in FIGS. 11A, 11B) or diametrically opposed (centroids 21A, 21B of the first and second diffusion window cross sectional planes 19A, 19B are diametrically opposed to each other as shown in FIG. 3A which is described herein.

FIG. 3A is a front schematic view of an exemplary air freshener 1 according to the present invention wherein the air freshener 1 is in a maximum intensity setting and the front housing part 12 is removed. The air freshener 1 of FIG. 3A comprises substantially the same components as the air freshener 1 of FIG. 1 except there are two diffusion windows 18A, 18B and corresponding first and second selector openings 22A, 22B.

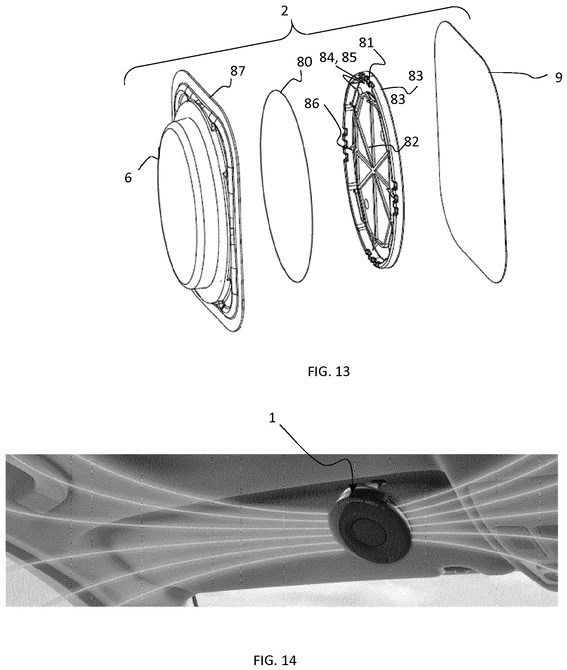

FIG. 3B is a schematic view of a first diffusion window cross sectional plane 19A extending across the first diffusion window 18A and FIG. 3C is a side schematic view of a second diffusion window cross sectional plane 19B extending across the second diffusion window 18B. Referring to FIG. 3B, the first diffusion window sectional plane 19A has a first diffusion window centroid 21A. Referring to FIG. 3C, the second diffusion window 18B has a second diffusion window centroid 21B. As shown in FIG. 3A, the air flow axis 200 intersects the first centroid 21A and the second centroid 21B defining a diffusion path 201 (not shown in FIG. 3A, but similar to the diffusion path of FIG. 2A) wherein the defined diffusion path 201 (not shown) is at least partially formed by the exterior evaporative surface 4 of the perfume containing body 2. Referring to FIGS. 3B, 3C, the first diffusion window cross sectional plane 19A and the second diffusion window cross sectional plane 19B each independently may have a cross sectional area having an aspect ratio (width 19W: length 19L) of at least 1:10, preferably from 1:10 to 37, from 1:10 to 1:1, from 1:8 to 1:2, from 1:8 to 1:4, or 1:8. For example when the first diffusion window cross sectional plane 19A or the second diffusion window cross sectional plane 19B may have an aspect ratio of 37 based on a length 19L of 56 mm and a width 19W of 1.5 mm. Aspect ratios in this range enable the housing 10 to be sized according to minimize an overall thickness of the air freshener 1 and improves aesthetic view when used on the sun visor in an automobile as shown in FIG. 14. It will be appreciated that the selector 20 is adjusted to uncover or cover the diffusion window cross sectional plane 19A as shown in FIGS. 6A to 10A and a percentage of the opening of the diffusion window cross sectional plane 19A may be calculated accordingly by multiplying the change in the length 19L with the width 19W.

The air freshener 1 of FIG. 1, 2A, 2B, 3A to 3C may comprise a diffusion path forming exterior evaporative surface cross sectional area 42A of 20 cm$^2$ to 50 cm$^2$, 20 cm$^2$ to 40 cm$^2$, 25 cm$^2$ to 30 cm$^2$, 27 cm$^2$ or different combinations of the upper and lower numerals described above or any combinations of any integer in the ranges listed above. At the minimum intensity setting, i.e. a smallest opening of the diffusion windows 18A, 18B, the first diffusion window cross sectional plane 19A and the second diffusion window cross sectional plane 19B may each independently have a cross sectional area of at least 0.9%, from 0.9% to 100%, from 1% to 80%, from 10% to 70%, from 1% to 15% relative to the diffusion path forming exterior evaporative surface cross sectional area 42A or different combinations of the upper and lower percentages described above or any combinations of any integer in the ranges listed above.

Table 1 below shows a relationship between the intensity setting (as a percentage of the overall selector opening/diffusion window 18A,18B), the cross-sectional area of the first/second diffusion window cross sectional plane 19A, 19B and the cross-sectional area as a percentage of the diffusion path forming exterior evaporative surface cross sectional area 42A. FIGS. 6A, 6B, 7A, 7B, 8A, 8B, 9A, 9B, 10A and 10B are schematic drawings illustrating examples of changing a position of the selector 20 to obtain a corresponding area change in a diffusion window cross sectional plane 19A/19B of the first or second diffusion window 18A, 18B of the air freshener 1 according to the present invention.

Table 1

| Inventive Sample Description | Cross-sectional area of Diffusion Window cross sectional plane 19A (cm$^2$) | % of diffusion path forming exterior evaporative surface cross sectional area 42A (An exemplary example is 27 cm$^2$) |
|---|---|---|
| Air Freshener of FIG. 6B - 5% opening (Minimum Intensity setting 60A) | 0.245 | 0.9 |
| Air Freshener of FIG. 7B - 10% opening (Intensity setting 60B) | 0.49 cm$^2$ | 1.8% |
| Air Freshener of FIG. 8B - 20% opening, (Intensity setting 60C) | 0.98 | 3.6 |
| Air Freshener of FIG. 9B - 50% opening, (Intensity setting 60D) | 2.45 | 9.1 |
| Air Freshener of FIG. 10B - 80% opening (Maximum intensity setting 60E) | 3.92 | 14.5 |

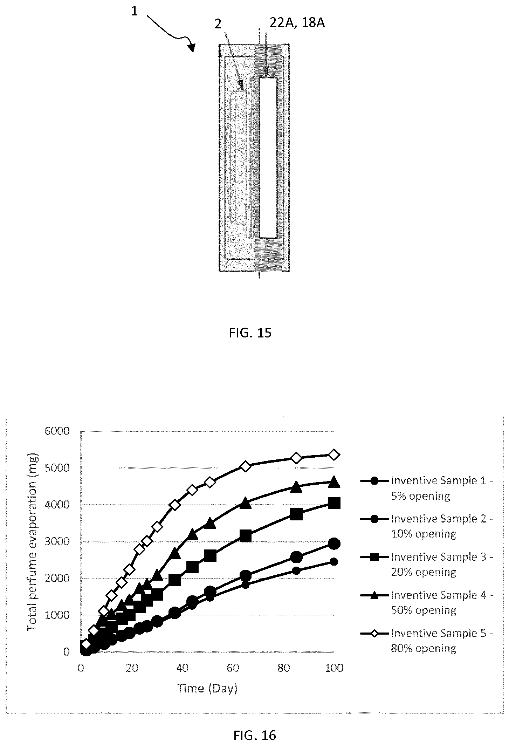

Specifically, having the first diffusion window cross sectional plane 19A and the second diffusion window cross sectional plane 19B each independently having a cross sectional area of at least 0.9%, 0.9% to 100%, 1% to 80%, 10% to 70%, 1% to 15% relative to the diffusion path forming exterior evaporative surface cross sectional area 42A at the minimum intensity setting 60A enables variation of scent intensity. The results are demonstrated in Example I with reference to the graph illustrated in FIG. 16. Further, the minimum cross-sectional area 201A of the defined diffusion path 201 may be from 0.2 cm$^2$ to 10 cm$^2$, from 3 cm$^2$ to 9 cm$^2$, from 4 cm$^2$ to 8 cm$^2$, from 5 cm$^2$ to 7 cm$^2$, or different combinations of the upper and lower numbers described above or combinations of any integer in the ranges listed above.

FIG. 4A shows a side schematic view of a variation of an air freshener 1 for varying scent intensity according to the present invention. The air freshener 1 of FIG. 4A comprises substantially the same components as the air freshener 1 of FIG. 3A except for the design of the housing 10, the perfume containing body 2 (amount of the volatile composition 8) and the size of a diffusion path forming exterior surface cross section area 42A of the perfume containing body 2. Specifically, when the volatile composition 8 is in an amount from about 2 ml to 4 ml, the diffusion path forming exterior surface cross sectional area 42A may be from 5 cm$^2$ to 15 cm$^2$, from 5 cm$^2$ to 10 cm$^2$, from 7 cm$^2$ to 10 cm$^2$ or different combinations of the upper and lower numerals described above or combinations of any integer in the ranges listed above. The air freshener 1 of FIG. 4A may have a length from 40 to 50 mm, a width from 40 to 50 mm and a thickness or depth from 25 mm to 40 mm from the front 61 of the container 6 to the rear housing part 14.

FIG. 4B is a side schematic view of the first diffusion window cross sectional plane 19A. The second diffusion window cross sectional plane 19B is symmetric to the first diffusion window cross sectional plane for ease of manufacturing and optimizing air flow through the housing 10 of the air freshener 1. The first diffusion window cross sectional plane 19A and the second diffusion window cross sectional plane 19B may each independently have a cross sectional area of at least 35%, from 35% to 150%, from 80% to 150%, from 100% to 150%, relative to the diffusion path forming exterior evaporative surface cross sectional area 19A/19B or different combinations of the upper and lower percentages described above or combinations of any integer in the ranges listed above.

Table 2 below shows a relationship between the intensity setting (as a percentage of the overall selector opening/diffusion window of the air freshener 1 of FIG. 4A), the cross-sectional area of the first/second diffusion window cross sectional plane 19A, 19B and the cross-sectional area as a percentage of the membrane area based on the perfume containing body 2.

Table 2

| Inventive Sample Description | Cross-sectional area of Diffusion Window cross sectional plane 19A (cm$^2$) | % of diffusion path forming exterior evaporative surface cross sectional area 42A (2.7 cm$^2$) |
|---|---|---|
| Air Freshener 1 of FIG.4A (Maximum Intensity Setting, 100% opening) | 10 | 143 |
| Air Freshener 1 of FIG. 4A (Middle Intensity Setting 34% opening) | 3.4 | 50 |
| Air Freshener 1 of FIG. 4A (Minimum Intensity Setting 26% opening) | 2.6 | 37 |

Specifically, having the first diffusion window cross sectional plane 19A and the second diffusion window cross sectional plane 19B each independently having have a cross sectional area of at least 35%, from 35% to 150%, from 80% to 150%, from 100% to 150%, relative to the diffusion path forming exterior evaporative surface cross sectional area 19A/19B relative to the diffusion path forming exterior evaporative surface cross sectional area 42A at the minimum intensity setting enables variation of scent intensity.

The cross-sectional area of the defined diffusion path 201 may be from 0.2 cm$^2$ to 10 cm$^2$, 3 cm$^2$ to 9 cm$^2$, 4 cm$^2$ to 8 cm$^2$, from 5 cm$^2$ to 7 cm$^2$ or any combinations of integers therebetween.

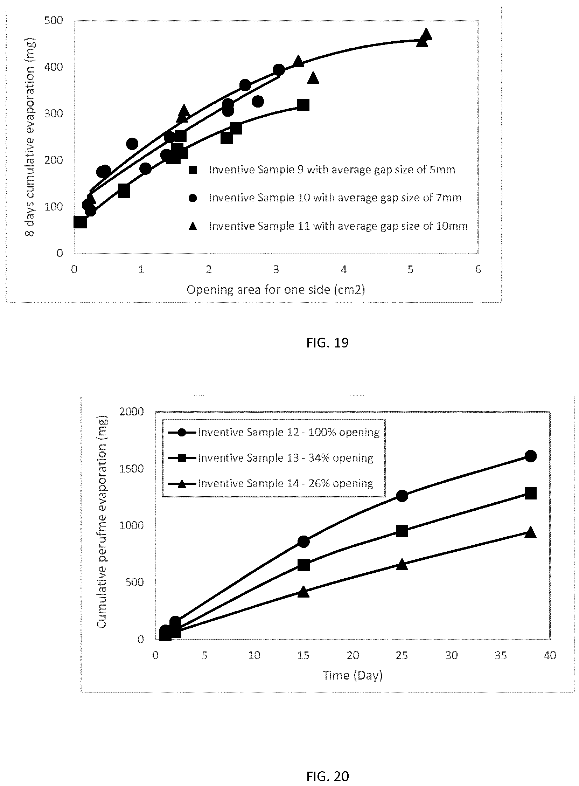

A technical effect of configuring the housing 10 and the diffusion window cross sectional plane 19A, 19B as described hereinbefore is that it provides an air freshener 1 capable of varying scent intensity. The results are demonstrated in Example VI with reference to the graph illustrated in FIG. 20.

The front housing part 12 may be integral with the rear housing part 14 to form a unitary housing as shown in FIG. 1. The front housing part 12 may also be a separate piece from the rear housing part 14 such that the housing 10 forms a two-piece structure as shown in FIG. 5. For the purposes of this disclosure, but without intending to limit the scope of the invention, the housing 10 is described as a two-piece structure.

FIG. 5 is a perspective view of components of an exemplary dispensing device 30 for an air freshener 1 according to the present invention. Specifically, the dispensing device 30 has substantially the same components as the dispensing device 30 of the air freshener 1 of FIG. 1 except that the housing shroud 16 has two diffusion windows 18 and the selector 20 has two corresponding selector openings 22. The housing shroud 16 may comprise a first shroud section 50 and a second shroud section 52 opposing the first shroud section 20, wherein the first and second shroud sections 50, 52 are disposed around at least part of the front housing part 12 which is removed to better illustrate the features. Specifically, the first shroud section 50 comprises a first diffusion window 18A and the second shroud section 52 comprises a second diffusion window 18B.

The first housing part 12 may also comprise indicia 122 corresponding to the range of positions of the selector 20 and support posts 124 extending away from the first housing part 12 for supporting and/or positioning the perfume containing body 2 within the housing 10. The selector 20 may include a selector planar portion 23 for receiving the front housing part 12 and a selector sleeve 24. The sleeve 24 is disposed on the planar portion 23 and extends away from the planar portion 23. The selector 20 may further comprise a digitally engageable protrusion 25 disposed on a sleeve outer surface 26 to facilitate single finger selection of a desired scent intensity by the user. The selector sleeve 24 has a sleeve cross-sectional area sized and shaped to receive the housing shroud 16. In FIG. 5, the selector 20 has a circular annular shape. However, the selector 20 may have a circular semi-annular shape, a hexagonal shape, a rectangular shape or any other shape suitable for adjusting a position of the selector 20 about the housing 10.

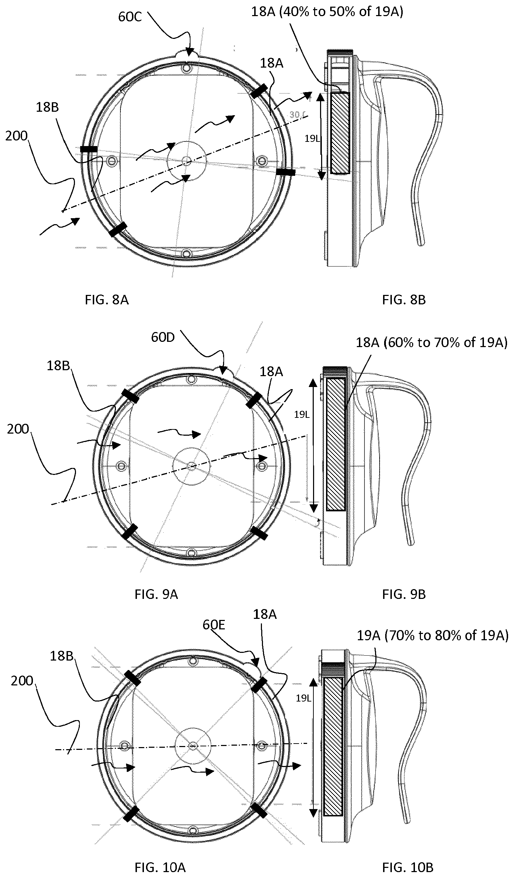

The housing shroud 16 may be integral with the rear housing part 14 to form a unitary part as shown in FIG. 5 to minimize number of parts for ease of assembly of the air freshener 1. The rear housing part 14 may include a rear wall 140 from which the shroud inner wall 160 of the housing shroud 16 extends. The rear wall 140 may be a solid wall as shown in FIG. 5 or comprise an opening such as shown in FIG. 10.

Referring to FIG. 5, the shroud inner wall 160 has open wall sections 164 and non-open wall sections 166 formed therein which upon assembly of the front housing part 12 to the rear housing part 14 define a first diffusion window 18 on a first shroud section 50 and a second diffusion window 18 on a second shroud section 52. Each of the open wall sections 164 and non-open wall sections 166 may be defined by varying a depth of the inner wall 160 with respect to a shroud bottom edge 168.

As shown in FIG. 5, the first diffusion window 18A and the second diffusion window 18B each independently is a single through hole upon assembly. However, the first diffusion window 18A or second diffusion window 18B each independently may comprise from 1 to 5, from 1 to 3, or from 1 to 2 through holes. The scent intensity selector 20 may comprise first and second selector openings 22 sized and shaped to align with the first and second diffusion windows 18A, 18B.

To explain the way the scent intensity selector 20 and the first and second diffusion windows 18 works to provide scent intensity variation according to the present invention, it is helpful to understand how a vapor release of volatile components in the perfume containing body 2 is generated. A method of varying scent intensity according to the present invention is described with reference to FIGS. 6A, 6B, 7A, 7B, 8A, 8B, 9A, 9B, 10A and 10B which are schematic drawings illustrating examples of changing a position of the selector 20 to obtain a corresponding area change in a diffusion window cross sectional plane 19A/19B of the first or second diffusion window 18A, 18B thereby changing a cumulative vapor release of the volatile composition.

In FIGS. 6A to 10B, the air freshener 1 has the front housing part 12 removed and is in a substantially vertical orientation. The perfume containing body 2 may be contained within the housing 10 in fluid communication with the first diffusion window 18A and the second diffusion window 18B, wherein the perfume containing body 2 comprises an exterior evaporative surface 4 described hereinbefore. Only diffusion window 18A is shown.

FIG. 6A is front view of a partial assembly of an exemplary air freshener 1 with the selector 20 in a first position corresponding to a first or minimum intensity setting 60A of the air freshener 1. FIG. 6B is a side section view of the air freshener 1 of FIG. 6A. When the selector 20 is rotated in a clockwise direction from a closed position through an angle relative to the center longitudinal axis 100 into the minimum intensity setting 60A, the selector opening 22A, 22B partially uncovers the first and second diffusion windows 18A, 18B such the size of the first and second diffusion windows 18A, 18B is approximately 1% to 10% of an individual cross sectional area of the respective diffusion window cross sectional plane 19A, 19B.

For illustrative purposes, the housing 10 and the selector 20 have a circular shape and the selector 20 is rotatably attached to the side of the housing 10 for adjusting a position of the selector 20 relative to the front housing part 12 are illustrated, but the present invention may be applied to any air freshener and is especially applicable to symmetrically shaped air fresheners or air fresheners with symmetrically shaped front housing parts. For example, the housing shroud 16 may be a rectangular shape such as shown in FIGS. 11A, 11B and comprises a length, wherein the scent intensity selector 20 is laterally slidable along the length of the housing shroud 16 in a direction parallel to the center longitudinal axis 100.

An advantage of having a rotatable scent intensity selector 20 with opposing selector openings 22A, 22A is that at the minimum intensity setting 60A, the length of the air flow path along the air flow axis 200 is along a diagonal of the diffusion path forming evaporative surface 42, i.e. the longest length. Accordingly, more volatile components of the volatile composition 8 may be evaporated and carried through air flow within the housing interior and through the diffusion windows 18A, 18B at the minimum intensity setting 60A to provide an effective amount of volatile composition 8 delivered to the interior space.

FIG. 7A is a front view of a partial assembly of the air freshener 1 of FIG. 6A with the selector 20 in a second position corresponding to a second scent intensity setting 60B of the air freshener 1. As the size of the first and second diffusion windows 18A, 18B individually is increased to approximately 15% to 30% of an individual cross-sectional area of the respective diffusion window cross sectional plane 19A, 19B, more air flow relative to air flow of FIG. 6A passes along the air flow axis 200 thereby increasing the diffusion of the volatile composition 8.

FIG. 8A is a front view of a partial assembly of the air freshener 1 of FIG. 6A with the scent intensity selector 20 in a third position corresponding to a third scent intensity setting 60C of the air freshener 1. FIG. 8B is a side section view of the air freshener 1 of FIG.7A. Referring to FIG. 8B, the size of the first and second diffusion windows 18A, 18B individually is approximately 40% to 50% of an individual cross-sectional area of the respective diffusion window cross sectional plane 19A, 19B.

FIG. 9A is a front view of a partial assembly of the air freshener 1 of FIG. 6A with the scent intensity selector 20 in a fourth position corresponding to a fourth scent intensity setting 60D of the air freshener 1. FIG. 9B is a side section view of the air freshener 1 of FIG.9A. Referring to FIG. 9B, the size of the first and second diffusion windows 18A, 18B individually is approximately 60% to 70% of an individual cross-sectional area of the respective diffusion window cross sectional plane 19A, 19B.

FIG. 10A is a front view of a partial assembly of the air freshener 1 of FIG. 6A with the scent intensity selector 20 in a fifth position corresponding to a fifth scent intensity setting 60E of the air freshener 1. FIG. 10B is a side section view of the air freshener 1 of FIG.10A. Referring to FIG. 10B, the size of the first and second diffusion windows 18A, 18B individually is approximately 70% to 80% of an individual cross-sectional area of the respective diffusion window cross sectional plane 19A, 19B. The fifth scent intensity setting 60E may correspond to a maximum intensity setting of the air freshener 1.

FIG. 12 is a perspective view of components of an exemplary dispensing device 30 for an air freshener 1 according to the present invention. Referring to FIG. 12, the dispensing device 30 has substantially the same components as the dispensing device 30 of FIG. 5 except that the dispensing device 30 further comprises an actuator 70 movable relative to the housing 10. The actuator 70 may be a push button 70 movable within an opening 126 of the front housing part 12. The push button 70 may comprise a button body 72 and a button cap 74 attached to the button body 72 to provide a contact area for the user to apply pressure to the push button 70 with a single finger. The rear housing part 14 differs from the rear housing part 14 of FIG. 5 in that there is a rear inner wall 142 disposed centrally in the rear wall 140 to define a rear opening for receiving a container 8 of the perfume containing body 2.

Further, the air freshener 1 may comprise a mounting portion 80 operably attached to the rear housing part 14 for attaching the air freshener 1 to an interior surface in an interior space. The interior space may be an automobile interior space. The interior surface in the automobile interior space may selected from the group consisting of: automobile sun visor, automobile seat rear pocket, automobile ceiling grab handle, car door pocket, more preferably, the interior surface in the automobile interior space is an automobile sun visor.

Referring to FIG. 12, the mounting portion 80 may comprise a resilient clip 90 disposed adjacent the rear housing part in a pre-mounting state in which it does not store energy (such as shown in FIG. 12) and arranged to exert a force on the interior surface upon attaching the mounting portion to the interior surface in a post-mounting state. The resilient clip 90 is adapted to flex to enable insertion of the mounting portion 80 to the interior surface and to exert a compression force on the interior surface after insertion. Specifically, the resilient clip 90 is adapted to enable an insertion force sufficient for securing the air freshener in use without damaging the interior surface. To increase a flexibility of the mounting portion 80 and/or for displaying a level or amount of the volatile composition 8 in the perfume containing body 2, the resilient clip 90 may comprise a slot 92 disposed centrally within the resilient clip 90.

Upon assembly of the perfume containing body 2 to the rear housing part 14, the rear wall 140 and the container 8 define a solid wall so as to minimize or prevent air flow through the rear housing part 14. The membrane 6 of the perfume containing body 2 is facing away from the rear housing part 14 so that when the first housing part 12 is assembled to the rear housing part 14 and the scent intensity selector 20, the push button 70 is disposed adjacent to the membrane 6 such that depression of the push button 70 activates release of a volatile composition from the container 8.

FIG. 13 is a perspective view of components of an exemplary perfume containing body 2 for an air freshener 1 according to the present invention. When the volatile composition 8 is a liquid perfume composition, the container 6 of the perfume containing body 2 may comprise a reservoir (not shown) and a rupturable substrate 80 sealably attached to and covering the reservoir to prevent the volatile composition 8 from being released until the air freshener 1 is activated. The rupturable substrate 80 may be ruptured to release the volatile composition 8 by actuating a rupture mechanism 81 positioned adjacent to the rupturable substrate 80.

The rupture mechanism 81 comprises a movable member 82 movably attached to an outer frame 83 by a resilient member 84. The resilient member 84 may be formed of one or more springs 85. One or more rupture elements 86 are arranged within the rupture mechanism 81 to puncture holes in the rupturable substrate 80. The rupture element 86 may be a pin. The membrane 9 may be sealably attached to a flange 87 located at the periphery of the container 6. The membrane 9 encloses the container 10, the volatile composition 8, the rupturable substrate 80, and the rupture mechanism 81. The membrane 9 may be configured to flex when a pressure or an actuation force is applied on the membrane 9 through the push button 70.

FIG. 14 is a front perspective view of an air freshener 1 according to the present invention in use in an automotive interior space wherein the air freshener 1 is mounted to an automotive sun visor. Mounting the air freshener 1 to the automotive sun visor enables air freshening of the automotive interior space without placing the air freshener on the dashboard surface which may obstruct view of the windshield. Further, as the volatile composition 8 is evaporated to the environment in the interior space through the sides of the air freshener 1, diffusion of the volatile composition to other areas in the automotive interior space may be achieved in a non-energized manner relying on the passive air ventilation in the automotive interior space. The air freshener 1 may be mounted to the automotive sun visor through the mounting portion 80 as shown in FIG. 12.

The air freshener 1 of the present invention may comprise a perfume containing body 2 having a liquid phase or a solid phase of a volatile composition 8. As the air freshener 1 described hereinbefore is non-energized, the volatile composition 8 may be formulated for use in non-energized vapor phase systems and the vapor pressure (hereinafter "VP") of one or more components of the volatile composition 8 may be at least 0.001 torr, from 0.001 to 10 torr, from 0.01 to 0.3 torr, 0.1 to 0.3 torr, or different combinations of the upper and lower percentages described above or combinations of any integer in the ranges listed above, measured at 25 degrees Celsius. The volatile composition may include perfume raw materials that solely provide a hedonic benefit (i.e. perfume raw materials which do not prevent mold yet provide a pleasant fragrance). The volatile composition 8 may be comprised in a cartridge 2 as shown in FIG. 13 according to the present invention. For the purposes of illustrating the present invention in detail, the invention is described below in connection with automobiles. However, it will be appreciated that the invention may be implemented in any interior occupancy space employing an air ventilation system.

The following examples are intended to more fully illustrate the present invention and are not to be construed as limitations of the present invention since many variations thereof are possible without departing from the scope of the present invention. All parts, percentages and ratios used herein are expressed as percent weight unless otherwise specified.

EXAMPLES

In order to determine the efficacy of the air freshener 1 in varying scent intensity, one would look at the cumulative weight loss and time average vapor release rate when the scent intensity dial is at the lowest setting to allow a narrow defined diffusion path across the membrane through the housing out to the external environment of the air freshener 1 and when the scent intensity dial is at the highest setting for a widest defined diffusion path across the membrane through the housing out to the external environment of the air freshener 1.

Test equipment/materials and test dispenser samples are first described under Materials, then the Test Method is provided, and lastly results are discussed. Data is provided demonstrating the air fresheners of the present invention having improved scent intensity control and achieves scent intensity variation.

MATERIALS

For the test methods/calculations described hereinafter, any volatile composition suitable for use in non-energized air fresheners or vapor phase systems may be employed. For illustrative purposes as well as for the subsequent examples, the volatile composition is a standard perfume composition as shown in the formulation of Table 3 below. The volatile composition, however, may constitute any number of materials suitable for air freshening.

Table 3

| Vapor Pressure 25°C Low | Vapor Pressure 25°C High | Perfume Composition 1 Components Wt Percentage | Perfume Composition 2 Components Wt Percentage |
|---|---|---|---|
| 0.00001 | 0.00 | 0.932 | 0.078 |
| 0.001 | 0.01 | 13.992 | 16.833 |
| 0.01 | 0.1 | 38.505 | 30.538 |
| 0.1 | 0.3 | 25.969 | 29.028 |
| 0.3 | 10 | 20.602 | 23.523 |
| | Total | 100 | 100 |

The volatile composition is contained in a perfume containing body 2, i.e. a volatile composition cartridge as shown in FIG. 13 (hereinafter "cartridge"). For calculation of the values detailed herein, one requires the following items:

1. Balance (Scale: Ohaus AA210 S/N 11131122540) or equivalent.
2. Housing of the present invention including a first and second wall
3. Volatile composition cartridges of FIG. 13 containing 6.5 ml of volatile composition of Table 3. (If adding a perfume composition by weight, multiply measured density by 6.5 ml to obtain the accurate fill weight.)
4. Volatile composition cartridges of FIG. 4A containing 2.4 ml of volatile composition of Table 3.
5. Housing 10 of FIG. 4A having the cross-sectional area of the first and second diffusion window cross sectional plane 19A, 19B based on Table 2 and overall dimensions of the cross-sectional area is as shown in FIG. 4B which is a cross-section schematic view of first and second diffusion window cross sectional plane 19A, 19B.
6. Dispensing devices of FIG. 12 (the cross-sectional area of the first and second diffusion window cross sectional plane 19A, 19B is based on Table 1) - Overall Dimensions is 86 mm (diameter) and 50 mm (depth/thickness including mounting portion) or 25 mm (depth of housing excluding the mounting portion).
7. Dispensing devices of FIG. 15 (the cross-sectional area of the first and second diffusion window cross sectional plane 19A, 19B is based on Table 1) - Overall Dimensions is 71.5 mm (Length) by 56 mm (Width) by 20 mm (Depth, Thickness).
8. 3M Scotch Weld Applicator TC and glue, #3797-TC or equivalent.
9. Evaporation rack or equivalent open tray (baker's) rack, covered at the top and shelves spaced at 15cm or more.
10. Room to accommodate evaporating rack with the following measurements, air flow, temperature / relative humidity or equivalent:

a) Laboratory Dimensions: 9.9 m long x 1.8 m wide x 1.8 m high or 49 $m^3$ (32 feet 4 inches long x 72 inches wide x 108 inches high or 1,730 $ft^3$)

b) Air Flow (Intake and Exhaust)

- Normal Mode: Average Intake Supply: 2.94 $m^3$/min (103.75 $ft^3$/min) $\pm$ 6%
- Average Exhaust: 4.23 $m^3$/min (149.25 $ft^3$/min) $\pm$ 6%
- Difference results in negative air pressure: -45.5
- Negative pressure indicates that air supply to laboratory and from an adjacent hallway or room is exhausted through the ventilation system.

c) Temperature and % Relative Humidity

- Average Temperature: 23° $\pm$ 2°C
- Average % Relative Humidity: 45% + 0.5%

TEST METHODS/CALCULATIONS

A. Test Method for determination of Cumulative Weight Loss and Time Average Vapor Release Rate

This test method is to determine a cumulative weight loss and time average vapor release rate of an inventive air freshener comprising a volatile composition.

1. Load the cartridge with the volatile composition in such a way as to provide a sealed cartridge that is not yet wetted. For instance, one may pierce the cartridge by cutting in it a hole that allows for insertion of a 18 gauge needle.
2. Fill the cartridge with 6.5 ml of volatile composition. This is equivalent to 6175 mg of the test composition. The volume may need to be adjusted based on the density of the composition of interest.
3. Seal the insertion hole with hot melt adhesive.
4. Measure and record the weight of the cartridge to three significant figures.
5. Insert the cartridge into the housing of dispensing device and ensure that the cartridge is set correctly within the housing to ensure proper air flow therethrough.
6. Activate the cartridge to wet its membrane. Herein, such activation is achieved by pressing the membrane by hand so as to rupture the rupturable substrate within the cartridge. There may, however, be equivalent means of activation and wetting the membrane.
7. Pick a time and measure (in mg) and record the cartridge and the dispensing device (housing and selector) weight daily at the same time for at least thirty days and up to 100 days.
8. Determine the cumulative weight loss of the volatile composition as detailed earlier using the recorded times with their corresponding weights.
9. Calculate the time average vapor release rate of the volatile composition as detailed earlier using the recorded times and their corresponding weights.

B. Cumulative Weight Loss Calculation Method

A cumulative weight loss at a time after activation is calculated following formula (1);

$$\text{Cumulative Weight Loss at } T_x = Wcum_{Tx} = W_{Ti} - W_{Tx} \quad (1)$$

wherein
$W_{Ti}$ = initial weight of volatile composition (mg) prior to activation
$W_{Tx}$ = weight of volatile composition (mg) at a designated time (days) after activation

C. Average Vapor Release Rate Calculation Method

An average vapor release rate of the air freshener is calculated using formula (2);

$$\text{Time average vapor release rate, } V = \frac{W_1 - W_2}{(t_2 - t_1)} \quad (2)$$

wherein,

ti = a first time period after activation
$t_2$ = a second time period after activation
Wi = weight of the volatile composition at ti
$W_2$ = weight of the volatile composition at $t_2$.

D. Vapor Release Rate Ratio Calculation Method

A vapor release rate ratio is calculated using formula (3);

$$Vapor\ Release\ Ratio\ V_R = \frac{V_H}{V_L}\ (3)$$

wherein,
$V_H$ = Vapor release rate (mg/h) of the air freshener at the highest setting of the scent intensity selector
$V_L$ = Vapor release rate (mg/h) of the air freshener at the lowest setting of the scent intensity selector

Example I

In this example, inventive air fresheners based on the dispensing device of FIG. 15, cartridge of FIG. 13 and the volatile composition of Table 3 ("Inventive Air Freshener Samples 1 to 5) are evaluated according to the Cumulative Weight Loss Test Method described hereinbefore under Test methods. For efficiency in generating the data, the inventive air fresheners are prepared such that each of the inventive air fresheners are adapted to represent one scent intensity setting only.

Table 4 below shows a cumulative weight loss of volatile composition from inventive air fresheners based on the dispensing device of FIG. 15. FIG. 16 is a corresponding graph of Table 4.

Table 4

| Time (Day) | Cumulative weight loss (mg) | | | | |
|---|---|---|---|---|---|
| | Inventive Sample 1 with 5% of each diffusion window 18A, 18B uncovered ("5% opening") | Inventive Sample 2 with 10% of each diffusion window 18A, 18B uncovered ("10% opening") | Inventive Sample 3 with 20% of each diffusion window 18A, 18B uncovered ("20% opening") | Inventive Sample 4 with 50% of each diffusion window 18A, 18B uncovered ("50% opening") | Inventive Sample 5 with 80% of each diffusion window 18A, 18B uncovered ("80% opening") |
| 0 | 0 | 0 | 0 | 0 | 0 |
| 2 | 50 | 94 | 164 | 223 | 214 |
| 5 | 130 | 159 | 323 | 514 | 586 |
| 9 | 229 | 247 | 503 | 859 | 1107 |
| 12 | 345 | 350 | 682 | 1029 | 1535 |
| 16 | 447 | 456 | 914 | 1279 | 1891 |
| 19 | 524 | 515 | 1010 | 1426 | 2242 |
| 23 | 645 | 615 | 1237 | 1731 | 2791 |
| 26 | 704 | 681 | 1400 | 1844 | 3011 |
| 30 | 837 | 798 | 1567 | 2103 | 3406 |
| 37 | 1072 | 1000 | 1954 | 2699 | 3997 |
| 44 | 1381 | 1262 | 2315 | 3202 | 4399 |
| 51 | 1641 | 1482 | 2621 | 3509 | 4614 |
| 65 | 2075 | 1832 | 3164 | 4061 | 5047 |
| 85 | 2583 | 2209 | 3751 | 4493 | 5269 |

(continued)

| Time (Day) | Cumulative weight loss (mg) | | | | |
|---|---|---|---|---|---|
| | Inventive Sample 1 with 5% of each diffusion window 18A, 18B uncovered ("5% opening") | Inventive Sample 2 with 10% of each diffusion window 18A, 18B uncovered ("10% opening") | Inventive Sample 3 with 20% of each diffusion window 18A, 18B uncovered ("20% opening") | Inventive Sample 4 with 50% of each diffusion window 18A, 18B uncovered ("50% opening") | Inventive Sample 5 with 80% of each diffusion window 18A, 18B uncovered ("80% opening") |
| 100 | 2950 | 2454 | 4052 | 4630 | 5364 |

Certain variations in the results of Table 4 (such as for example a lower weight loss detected in Day 19 at 10% relative to a weight loss detected in Day 19 at 5%) may be due to slight differences in positioning of the inventive samples in the room in which the experiment/test is conducted to obtain results in an efficient manner. Referring to the corresponding graph of Table 4 in FIG. 16, there is a difference in cumulative perfume evaporation between the inventive air freshener sample at all specified scent intensity settings, i.e. a maximum scent intensity setting of 80%, a minimum scent intensity setting of 5% and scent intensity settings therebetween of 10%, 20%, 50%.

Table 5 below shows an average vapor release rate of the inventive air fresheners calculated based on the cumulative weight loss results of Table 4.

Table 5

| Time (Day) | Average vapor release rate (mg/h) | | | | |
|---|---|---|---|---|---|
| | 5% Opening | 10% Opening | 20% Opening | 50% Opening | 80% Opening |
| 0 | 0 | 0 | 0 | 0 | 0 |
| 2 | 1.04 | 1.96 | 3.42 | 4.65 | 4.46 |
| 5 | 1.11 | 0.90 | 2.21 | 4.04 | 5.17 |
| 9 | 1.03 | 0.92 | 1.88 | 3.59 | 5.43 |
| 12 | 1.61 | 1.43 | 2.49 | 2.36 | 5.94 |
| 16 | 1.06 | 1.10 | 2.42 | 2.60 | 3.71 |
| 19 | 1.07 | 0.82 | 1.33 | 2.04 | 4.87 |
| 23 | 1.26 | 1.04 | 2.36 | 3.18 | 5.72 |
| 26 | 0.82 | 0.92 | 2.26 | 1.57 | 3.06 |
| 30 | 1.39 | 1.22 | 1.74 | 2.70 | 4.11 |
| 37 | 1.40 | 1.20 | 2.30 | 3.55 | 3.52 |
| 44 | 1.84 | 1.56 | 2.15 | 2.99 | 2.39 |
| 51 | 1.55 | 1.31 | 1.82 | 1.83 | 1.28 |
| 65 | 1.29 | 1.04 | 1.62 | 1.64 | 1.29 |
| 85 | 1.06 | 0.79 | 1.22 | 0.90 | 0.46 |
| 100 | 1.02 | 0.68 | 0.84 | 0.38 | 0.26 |

The above results show that a diffusion window 18 having a cross sectional area of at least 0.9% of the diffusion path evaporative surface cross sectional area 42A enables a weight loss corresponding to a perceivable scent by users at the minimum scent intensity setting. The above results also show that the vapor release rate at 80% opening is lower than the vapor release rate at 5% opening. It will be appreciated that as perfume evaporates at a higher rate in the initial days and therefore there is less perfume or volatile composition remaining in the cartridge for vaporization from Day 51 to Day 100.

Example II

In this example, inventive air fresheners based on the dispensing device of FIG. 15 (Inventive Samples 1, 2, 3) and inventive air fresheners based on the dispensing device of FIG. 12 (Inventive Samples 6, 7, 8) are evaluated according to the Cumulative Weight Loss Test Method described hereinbefore under Test methods. Cartridges of FIG. 13 containing the volatile composition of Table 3 are used. For efficiency in generating the data, the inventive air fresheners are prepared such that each of the inventive air fresheners are adapted to represent one scent intensity setting only.

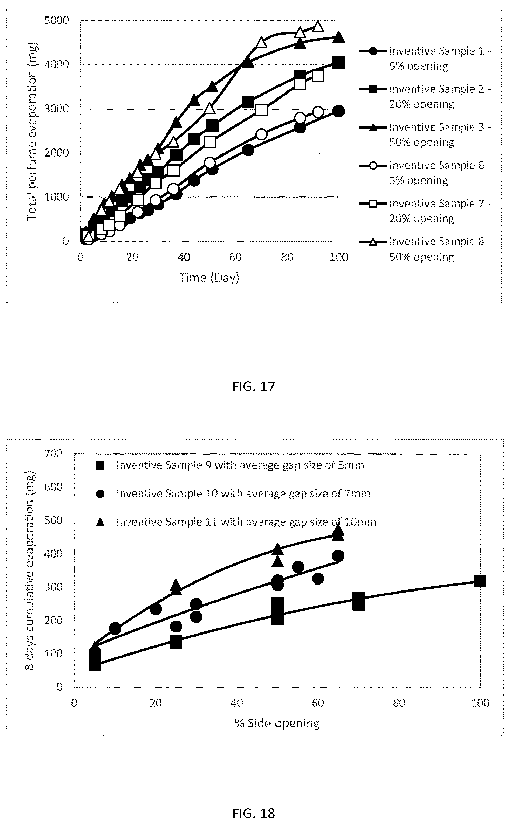

As shown in the graph of FIG. 17, at a first intensity setting of the respective inventive air fresheners, i.e. when the diffusion windows 18A, 18B are opened or uncovered at 5% of the diffusion window cross sectional plane for inventive air fresheners based on the dispensing device of FIG. 12 and the dispensing device of FIG. 15 respectively, the corresponding perfume evaporation or cumulative weight loss curves are substantially the same or have little differences from Day 0 to Day 90. At a second scent intensity setting, i.e. when the diffusion windows 18A, 18B are opened at 20%, the perfume evaporation curves representing the dispensing devices of FIG. 12, FIG. 15 are also substantially similar. At a third scent intensity setting, i.e. when the diffusion windows 18A, 18B are opened or uncovered at 50%, the perfume evaporation curves representing the dispensing devices of FIG. 12, FIG. 15 are also substantially similar.

Without wishing to be bound by theory and in view of comparison of and similarity between the results at the same scent intensity settings as illustrated in the graph of FIG. 17, the cumulative weight loss results of the inventive air fresheners having dispensing devices of FIG. 12 is likely to correspond substantially to the results of the inventive air fresheners having dispensing devices of FIG. 15 at all the scent intensity settings shown in Table 4 with reference to FIG. 16. Specifically, the results show that a circular housing 10 and a rectangular or elongate housing 10 demonstrate the same technical results and scent variation advantage based on the air freshener 1 according to the present invention having a diffusion window having a cross sectional area of at least 0.9% of the diffusion path evaporative surface cross sectional area 42A.

Example III

In this example, inventive air fresheners based on the dispensing device of FIG. 15 are adapted to demonstrate an effect of a cumulative weight loss of the volatile composition relative to a percentage of the diffusion window at different gaps between the diffusion path evaporative surface cross sectional area 42A and the inner surface of the front housing part 12. Table 6 below shows an average cumulative weight loss of the inventive air fresheners at different gaps ("Inventive Samples 9, 10, 11") described hereinbefore calculated as a function of the percentage of the diffusion window that is uncovered or open.

Table 6

| Inventive Sample 9 with Average gap size 5mm | | | Inventive Sample 10 with Average gap size 7 mm | | | Inventive Sample 11 with Average gap size 10 mm | | |
|---|---|---|---|---|---|---|---|---|
| % opening | Area (mm2) | Day 8 CWL (mg) | % opening | Area (mm2) | Day 8 CWL (mg) | % opening | Area (mm2) | Day 8 CWL (mg) |
| 5 | 8 | 68 | 5 | 24 | 93 | 5 | 24 | 120 |
| 5 | 10 | 68 | 5 | 20 | 105 | 25 | 160 | 294 |
| 25 | 74 | 133 | 10 | 42 | 176 | 25 | 163 | 308 |
| 25 | 74 | 138 | 10 | 46 | 178 | 50 | 333 | 414 |
| 50 | 145 | 206 | 20 | 86 | 236 | 50 | 355 | 378 |
| 50 | 149 | 206 | 25 | 106 | 183 | 65 | 517 | 456 |
| 50 | 140 | 210 | 30 | 137 | 212 | 65 | 523 | 472 |
| 50 | 153 | 225 | 30 | 142 | 250 | | | |
| 50 | 160 | 217 | 50 | 228 | 321 | | | |
| 50 | 158 | 253 | 50 | 228 | 307 | | | |
| 70 | 226 | 249 | 55 | 254 | 362 | | | |
| 70 | 240 | 269 | 60 | 273 | 327 | | | |

(continued)

| Inventive Sample 9 with Average gap size 5mm | | | Inventive Sample 10 with Average gap size 7 mm | | | Inventive Sample 11 with Average gap size 10 mm | | |
|---|---|---|---|---|---|---|---|---|
| % opening | Area (mm2) | Day 8 CWL (mg) | % opening | Area (mm2) | Day 8 CWL (mg) | % opening | Area (mm2) | Day 8 CWL (mg) |
| 100 | 340 | 320 | 65 | 304 | 395 | | | |

With reference to FIG. 18, the results show that providing a gap of about 7mm, about 5 mm, or about 10 mm enable scent variation over different percentages of the diffusion window that is uncovered. FIG. 19 is a corresponding graph of FIG. 18 except that the percentage of the diffusion window uncovered is expressed as a function of the diffusion path evaporative surface cross sectional area 42A. The results of FIG. 19 show that perfume evaporation amount is dependent on the diffusion path evaporative surface cross sectional area 42A and a larger opening area will provide a corresponding increase in the perfume evaporation for the above gap ranges.

Example IV

In this example, inventive air fresheners based on the air freshener of FIG. 4 and the volatile compositions of Table 3 ("Inventive Samples 12, 13, 14") are evaluated according to the Cumulative Weight Loss Test Method described hereinbefore under Test methods. For efficiency in generating the data, the inventive air fresheners are prepared such that each of the inventive air fresheners are adapted to represent one scent intensity setting only.
Table 7 below shows a cumulative weight loss of volatile composition from inventive air fresheners based on the dispensing device of FIG. 4. FIG. 20 is a corresponding graph of Table 7.

Table 7

| Time (Day) | Total perfume evaporation (mg) | | |
|---|---|---|---|
| | Inventive Sample 12 (100% opening) | Inventive Sample 13 (34% opening) | Inventive Sample 14 (26% opening) |
| 0 | 0 | 0 | 0 |
| 1 | 71 | 41 | 39 |
| 2 | 145 | 85 | 69 |
| 15 | 800 | 658 | 422 |
| 25 | 1113 | 953 | 662 |
| 38 | 1444 | 1286 | 946 |

The results in Table 7 with reference to FIG. 20 show that inventive air fresheners based on a dispensing device of FIG. 4 having a diffusion window of a cross sectional area of at least 37% of the diffusion path evaporative surface cross sectional area 42 (2.7 cm$^2$) provides scent intensity variation for at least three different scent intensity settings.
Table 8 below shows an average vapor release rate of the inventive air fresheners calculated based on the cumulative weight loss results of Table 7.

Table 8

| Time (Day) | Perfume evaporation rate (mg/h) | | |
|---|---|---|---|
| | Inventive Sample 12 (100% opening") | Inventive Sample 13 (34% opening) | Inventive Sample 14 (26% opening) |
| 0 | 0 | 0 | 0 |
| 1 | 2.96 | 1.71 | 1.62 |
| 2 | 2.74 | 1.63 | 1.11 |
| 15 | 2.11 | 1.84 | 1.14 |

(continued)

| Time (Day) | Perfume evaporation rate (mg/h) | | |
|---|---|---|---|
| | Inventive Sample 12 (100% opening") | Inventive Sample 13 (34% opening) | Inventive Sample 14 (26% opening) |
| 25 | 1.32 | 1.24 | 1.01 |
| 38 | 1.08 | 1.09 | 0.93 |

The above results show different perfume evaporation rates at each of the above different opening sizes in the air freshener 1. This demonstrates that the air freshener 1 according to the present invention having the specified diffusion window cross sectional plane at the specified areas as shown in Table 2 enable scent variation in the air freshener 1.

Example IV- Inventive Air freshener having vapor release rate ratio 1.3

In this example, the calculations are based on the results in Table 9 below which show that an air freshener having a dispensing device of FIG. 15 and a scent intensity setting of 20% opening as the lowest setting and 80% opening as the highest setting. For a given perfume composition, based on the vapor release rate ratio test method described hereinbefore, the vapor release rate ratio is calculated two days from product activation is given by:

$$V_R = \frac{V_H}{V_L} = \frac{4.46}{3.42} = 1.3$$

Table 9

| Time (Day) | Perfume evaporation rate (mg/h) | |
|---|---|---|
| | Inventive Sample 2 - 20% opening | Inventive Sample 3 80% opening |
| 2 | 3.42 | 4.46 |

Example V - Inventive Air freshener having vapor release rate ratio of 75

In this example, the calculations are based on the results in Table 10 below for an air freshener having a dispensing device of FIG. 4. For a given perfume composition, the vapor release ratio based on one day from product activation is given by:

$$V_R = \frac{V_H}{V_L} = \frac{2.96}{0.04} = 75$$

Table 10

| Time (Day) | Perfume evaporation rate (mg/h) | | | |
|---|---|---|---|---|
| | Inventive Sample 12 (100% opening) | Inventive Sample 13 (34% opening) | Inventive Sample 14 (26% opening) | Inventive Sample 15 (covered with gaps at the joining of parts) |
| 1 | 2.96 | 1.71 | 1.62 | 0.04 |

Example VI - Inventive Air freshener having vapor release rate ratio of 4.3

In this example, the calculations are based on the results in Table 11 below for an air freshener having a dispensing device of FIG. 15. For a given perfume composition, the vapor release ratio of the air freshener two days from product activation is given by:

EP 3 682 910 B1

$$V_R = \frac{V_H}{V_L} = \frac{4.46}{1.04} = 4.3$$

Table 11

| Time (Day) | Perfume evaporation rate (mg/h) | | | | |
|---|---|---|---|---|---|
| | Inventive Sample 1 5% opening | Inventive Sample 2 10% opening | Inventive Sample 3 20% opening | Inventive Sample 4 50% opening | Inventive Sample 5 80% opening |
| 2 | 1.04 | 1.96 | 3.42 | 4.65 | 4.46 |

The above results in Examples IV, V and VI show that the air freshener according to the present invention can provide a vapor release rate ratio from 1.3 to 75 based on the test methods described hereinbefore.

An example is shown below:

A. An air freshener capable of varying scent intensity, the air freshener operably connectable to a mounting portion for releasably attaching the air freshener to an interior surface in an interior space, the air freshener comprising:

(a) a housing having a front housing part and a housing shroud, wherein the housing shroud circumscribes at least part of the front housing part to define a side of the housing;
(b) a center longitudinal axis extending along a length (L) of the front housing part and extending through a center of the front housing part;
(c) a diffusion window disposed in the housing shroud, the diffusion window having a diffusion window cross sectional plane extending across the diffusion window, wherein the diffusion window sectional plane has a diffusion window centroid;
(d) a perfume containing body contained within the housing in fluid communication with the diffusion window, wherein the perfume containing body comprises an exterior evaporative surface;
(e) an air flow axis intersecting the diffusion window centroid defining a diffusion path, wherein the defined diffusion path is at least partially formed by the exterior evaporative surface of the perfume containing body, wherein a diffusion path forming exterior evaporative surface is in fluid communication with the defined diffusion path and has a diffusion path forming exterior evaporative surface cross sectional area of 5 cm$^2$ to 100 cm$^2$;
(f) wherein the defined diffusion path comprises a minimum cross-sectional area of at least 0.2 cm$^2$ measured along the length of the air flow axis and at least along the length of the diffusion path forming exterior evaporative surface; and
(g) a scent intensity selector adjustably attached to the housing shroud for at least partially covering or uncovering the diffusion window.

B. The air freshener according to A, wherein a ratio of an average vapor release rate of the air freshener at a maximum scent intensity setting of the selector to an average vapor release rate of the air freshener at a minimum scent intensity setting of the selector is from about 1.3 to about 75.

C. The air freshener according to A, wherein the housing shroud comprises:

a first shroud section and a second shroud section opposing the first shroud section, wherein the first and second shroud sections are disposed around at least part of the front housing part;
wherein the first shroud section comprises a first diffusion window having a first diffusion window cross sectional plane extending across the first diffusion window, wherein the first diffusion window sectional plane has a first diffusion centroid;
wherein the second shroud section comprises a second diffusion window having a second diffusion window cross sectional plane extending across the second diffusion window, wherein the second diffusion window cross sectional plane has a second diffusion window centroid;
wherein the air flow axis intersects the first diffusion window centroid and the second diffusion window centroid defining a diffusion path therein between; wherein the defined diffusion path is at least partially formed by the exterior evaporative surface of the perfume containing body, wherein the diffusion path forming exterior evaporative surface is in fluid communication with the defined diffusion path wherein the scent intensity selector is adjustably attached to the housing shroud for at least partially covering or uncovering the first diffusion window

18

and second diffusion window.

D. The air freshener according to C, wherein the diffusion path forming exterior evaporative surface has a diffusion path forming exterior evaporative surface cross sectional area of 5 cm$^2$ to 15 cm$^2$, preferably 5 cm$^2$ to 10 cm$^2$, more preferably 7 cm$^2$ to 10 cm$^2$;
wherein the first diffusion window cross sectional plane and the second diffusion window cross sectional plane each independently have a cross sectional area of at least 35%, preferably 35% to 150%, more preferably 80% to 150%, even more preferably 100% to 150% relative to the diffusion path forming exterior evaporative surface cross sectional area.

E. The air freshener according to claim D, wherein the minimum cross-sectional area of the defined diffusion path is 0.2 cm$^2$ to 10 cm$^2$, preferably 3 cm$^2$ to 9 cm$^2$, more preferably from 4 cm$^2$ to 8 cm$^2$, even more preferably from 5 cm$^2$ to 7 cm$^2$.

F. The air freshener according to C, wherein the diffusion path forming exterior evaporative surface has a diffusion path forming exterior evaporative surface cross sectional area of 20 cm$^2$ to 50 cm$^2$, preferably 20 cm$^2$ to 40 cm$^2$, more preferably 25 cm$^2$ to 30 cm$^2$
wherein the first diffusion window cross sectional plane and the second diffusion window cross sectional plane each independently have a cross sectional area of at least 0.9%, preferably 0.9% to 100%, more preferably 1% to 80%, even more preferably 10% to 70%, yet even more preferably 1% to 15%, relative to the diffusion path forming exterior evaporative surface cross sectional area.

G. The air freshener according to claim F, wherein the minimum cross-sectional area of the defined diffusion path (201) is 0.2 cm$^2$ to 10 cm$^2$, preferably 3 cm$^2$ to 9 cm$^2$, more preferably from 4 cm$^2$ to 8 cm$^2$, even more preferably from 5 cm$^2$ to 7 cm$^2$.

H. The air freshener according to C, wherein the first and second diffusion windows are diametrically opposed, preferably the first and second diffusion windows are aligned with first and second selector openings of the scent intensity selector for symmetrically covering and uncovering the first diffusion window and the second diffusion window.

I. The air freshener according to C, wherein the first selector opening or second selector opening each independently comprises from 1 to 5, preferably from 1 to 3, more preferably from 1 to 2 through holes.

J. The air freshener according to A, wherein the housing shroud comprises a length, wherein the scent intensity selector is laterally slidable along the length of the housing shroud in a direction parallel to the center longitudinal axis.

K. The air freshener according to A, wherein the housing shroud comprises a circular shape, wherein the scent intensity selector is rotatable about a center axis of the housing shroud.

L. The air freshener according to K, wherein the scent intensity selector is rotatable through a range of rotation angles up to 90 degrees, preferably from 1 degree to 85 degrees, more preferably from 5 degrees to 80 degrees, even more preferably from 10 degrees to 75 degrees.

M. The air freshener according to A, wherein the scent intensity selector further comprises a digitally engageable protrusion to single handedly adjust the scent intensity selector.

N. The air freshener according to A, wherein the perfume containing body comprises a container containing a volatile composition and a membrane in fluid communication with the volatile composition, wherein the exterior surface of the membrane forms at least a portion of said exterior evaporative surface of the perfume containing body.

O. The air freshener according to N, wherein an actuator movable relative to the housing is disposed adjacent to the membrane such that moving the actuator activates release of the volatile composition from the perfume containing body.

P. The air freshener according to A, wherein the housing comprises a rear housing part and a mounting portion operably attachable to rear housing part for attaching the air freshener to an interior surface in an interior space, preferably the mounting portion comprises a resilient clip disposed adjacent the housing in a pre-mounting state in which the resilient clip does not store energy and arranged to exert a force on the interior surface upon attaching the resilient clip of the mounting portion to the interior surface in a mounting state, preferably the resilient clip and the housing form a unitary unit, more preferably, the resilient clip comprises plastic.

Q. An air freshener capable of varying scent intensity, the air freshener operably connectable to a mounting portion for releasably attaching the air freshener to an interior surface in an interior space, the air freshener comprising:

(a) a housing having a front housing part and a housing shroud, wherein the housing shroud circumscribes at least part of the front housing part to define a side of the housing;
(b) a center longitudinal axis extending along a length (L) of the front housing part and extending through a center of the front housing part;
(c) a diffusion window disposed in the housing shroud, the diffusion window having a diffusion window cross sectional plane extending across the diffusion window, wherein the diffusion window sectional plane has a diffusion window centroid;
(d) a perfume containing body contained within the housing in fluid communication with the diffusion window, wherein the perfume containing body comprises an exterior evaporative surface;
(e) an air flow axis intersecting the diffusion window centroid defining a diffusion path, wherein the defined diffusion path is at least partially formed by the exterior evaporative surface of the perfume containing body, wherein a diffusion path forming exterior evaporative surface is in fluid communication with the defined diffusion path and has a diffusion path forming exterior evaporative surface cross sectional area of 5 cm$^2$ to 100 cm$^2$;
(f) wherein the defined diffusion path comprises a minimum cross-sectional area of at least 0.2 cm$^2$ measured along the length of the air flow axis and at least along the length of the diffusion path forming exterior evaporative surface;
(g) a scent intensity selector adjustably attached to the housing shroud for at least partially covering or uncovering the diffusion window; and
(h) wherein a ratio of an average vapor release rate of the air freshener at a maximum scent intensity of the selector to an average vapor release rate of the air freshener at a minimum scent intensity setting of the selector is from about 1.3 to about 75, wherein the ratio is determined in accordance with the Vapor Release Rate Ratio Test Method specified herein.

R. A method of varying scent intensity in an interior space, the method comprising:

(i) providing an air freshener in an interior space, wherein the air freshener comprises:

(a) a housing having a front housing part and a housing shroud, wherein the housing shroud circumscribes at least part of the front housing part to define a side of the housing;
(b) a center longitudinal axis extending along a length (L) of the front housing part and extending through a center of the front housing part;
(c) a diffusion window disposed in the housing shroud, the diffusion window having a diffusion window cross sectional plane extending across the diffusion window, wherein the diffusion window sectional plane has a diffusion window centroid;
(d) a perfume containing body contained within the housing in fluid communication with the diffusion window, wherein the perfume containing body comprises an exterior evaporative surface;
(e) an air flow axis intersecting the diffusion window centroid defining a diffusion path, wherein the defined diffusion path is at least partially formed by the exterior evaporative surface of the perfume containing body, wherein a diffusion path forming exterior evaporative surface is in fluid communication with the defined diffusion path and has a diffusion path forming exterior evaporative surface cross sectional area of 5 cm$^2$ to 100 cm$^2$;
(f) wherein the defined diffusion path comprises a minimum cross-sectional area of at least 0.2 cm$^2$ measured along the length of the air flow axis and at least along the length of the diffusion path forming exterior evaporative surface; and
(g) a scent intensity selector adjustably attached to the side of the housing for at least partially covering or uncovering the diffusion window; and

(ii) adjusting the scent intensity selector relative to the center of the front housing part and along the side of the housing, wherein the scent intensity selector has a range of selector positions relative to the center of the front housing part; wherein a desired scent intensity setting is identifiable by a position of the scent intensity selector along the range of selector positions, preferably the scent intensity selector has at least three selector positions, more preferably, the scent intensity selector has five selector positions.

S. The method according to R, wherein providing the air freshener in the interior space comprises sliding the air freshener over an interior surface to attach the air freshener to the interior surface, preferably the interior space is an automobile interior space, and the interior surface is selected from the group consisting of: automobile sun visor, automobile seat rear pocket, automobile ceiling grab handle, car door pocket, more preferably the interior surface is an automobile sun visor.

T. A dispensing device for an air freshener, the device comprising:

(a) a housing having a front housing part and a housing shroud, wherein the housing shroud circumscribes at least part of the front housing part to define a side of the housing;
(b) a center longitudinal axis extending along a length (L) of the front housing part and extending through a center of the front housing part;
(c) a diffusion window disposed in the housing shroud, the diffusion window having a diffusion window cross sectional plane extending across the diffusion window, wherein the diffusion window sectional plane has a diffusion window centroid;
(d) wherein the housing is configured for containing a perfume containing body within the housing in fluid communication with the diffusion window, wherein the perfume containing body comprises an exterior evaporative surface;
(e) an air flow axis intersecting the diffusion window centroid defining a diffusion path, wherein the defined diffusion path is at least partially formed by the exterior evaporative surface of the perfume containing body, wherein a diffusion path forming exterior evaporative surface is in fluid communication with the defined diffusion path and has a diffusion path forming exterior evaporative surface cross sectional area of 5 cm2 to 100 cm2;
(f) wherein the defined diffusion path comprises a minimum cross-sectional area of at least 0.2 cm2 measured along the length of the air flow axis and at least along the length of the diffusion path forming exterior evaporative surface; and
(g) a scent intensity selector adjustably attached to the side of the housing for at least partially covering or uncovering the diffusion window.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1.  An air freshener (1) capable of varying scent intensity, the air freshener (1) operably connectable to a mounting portion for releasably attaching the air freshener (1) to an interior surface in an interior space, the air freshener (1) comprising:

(a) a housing (10) having a front housing part (12) and a housing shroud (16), wherein the housing shroud (16) circumscribes at least part of the front housing part (12) to define a side of the housing;
(b) a center longitudinal axis (100) extending along a length of the front housing part (12) and extending through a center of the front housing part (12);
(c) a diffusion window (18) disposed in the housing shroud (16), the diffusion window (18) having a diffusion window cross sectional plane (19) extending across the diffusion window (18), wherein the diffusion window sectional plane (19) has a diffusion window centroid (21);
(d) a perfume containing body (2) contained within the housing (10) in fluid communication with the diffusion window (18), wherein the perfume containing body (2) comprises an exterior evaporative surface (4);
(e) an air flow axis (200) intersecting the diffusion window centroid (21) defining a diffusion path (201), wherein the defined diffusion path (201) is at least partially formed by the exterior evaporative surface (4) of the perfume containing body (2), wherein a diffusion path forming exterior evaporative surface (42) is in fluid communication with the defined diffusion path (201) and has a diffusion path forming exterior evaporative surface cross sectional area (42A) of 5 cm2 to 100 cm2;
(f) wherein the defined diffusion path (201) comprises a minimum cross-sectional area (201A) of at least 0.2 cm2 measured along the length of the air flow axis (200) and at least along the length (42D) of the diffusion path forming exterior evaporative surface (42); and
(g) a scent intensity selector (20) adjustably attached to the side of the housing (10) for at least partially covering or uncovering the diffusion window (18).

2. The air freshener (1) according to claim 1, wherein a ratio of an average vapor release rate of the air freshener (1) at a maximum scent intensity setting of the selector (20) to an average vapor release rate of the air freshener (1) at a minimum scent intensity setting of the selector (20) is from about 1.3 to about 75.

3. The air freshener (1) according to claim 1 or claim 2, wherein the housing shroud (16) comprises:

a first shroud section (50A) and a second shroud section (50B) opposing the first shroud section (50A), wherein the first and second shroud sections (50A, 50B) are disposed around at least part of the front housing part (12); wherein the first shroud section (50A) comprises a first diffusion window (18A) having a first diffusion window cross sectional plane (19A) extending across the first diffusion window (18A), wherein the first diffusion window sectional plane (19A) has a first diffusion centroid (21A);
wherein the second shroud section (50B) comprises a second diffusion window (18B) having a second diffusion window cross sectional plane (19B) extending across the second diffusion window (18B), wherein the second diffusion window cross sectional plane (19B) has a second diffusion window centroid (21B);
wherein the air flow axis (200) intersects the first diffusion window centroid (21A) and the second diffusion window centroid (21B) defining a diffusion path (201) therein between; wherein the defined diffusion path (201) is at least partially formed by the exterior evaporative surface (4) of the perfume containing body (2), wherein the diffusion path forming exterior evaporative surface (42) is in fluid communication with the defined diffusion path (201) wherein the scent intensity selector (20) is adjustably attached to the housing shroud (16) for at least partially covering or uncovering the first diffusion window (18A) and the second diffusion window (18B).

4. The air freshener (1) according to claim 3, wherein the diffusion path forming exterior evaporative surface (42) has a diffusion path forming exterior evaporative surface cross sectional area (42A) of 5 cm$^2$ to 15 cm$^2$, preferably 5 cm$^2$ to 10 cm$^2$, more preferably 7 cm$^2$ to 10 cm$^2$;
wherein the first diffusion window cross sectional plane (19A) and the second diffusion window cross sectional plane (19B) each independently have a cross sectional area of at least 35%, preferably 35% to 150%, more preferably 80% to 150%, even more preferably 100% to 150% relative to the diffusion path forming exterior evaporative surface cross sectional area (42A).

5. The air freshener (1) according to claim 4, wherein the minimum cross-sectional area (201A) of the defined diffusion path (201) is 0.2 cm$^2$ to 10 cm$^2$, preferably 3 cm$^2$ to 9 cm$^2$, more preferably from 4 cm$^2$ to 8 cm$^2$, even more preferably from 5 cm$^2$ to 7 cm$^2$.

6. The air freshener (1) according to claim 3, wherein the diffusion path forming exterior evaporative surface (42) has a diffusion path forming exterior evaporative surface cross sectional area (42A) of 20 cm$^2$ to 50 cm$^2$, preferably 20 cm$^2$ to 40 cm$^2$, more preferably 25 cm$^2$ to 30 cm$^2$;
wherein the first diffusion window cross sectional plane (19A) and the second diffusion window cross sectional plane (19B) each independently have a cross sectional area of at least 0.9%, preferably 0.9% to 100%, more preferably 1% to 80%, even more preferably 10% to 70%, yet even more preferably 1% to 15% relative to the diffusion path forming exterior evaporative surface cross sectional area (42A).

7. The air freshener (1) according to claim 6, wherein the minimum cross-sectional area (201A) of the defined diffusion path (201) is 0.2 cm$^2$ to 10 cm$^2$, preferably 3 cm$^2$ to 9 cm$^2$, more preferably from 4 cm$^2$ to 8 cm$^2$, even more preferably from 5 cm$^2$ to 7 cm$^2$.

8. The air freshener (1) according to any one of claims 3 to 7, wherein the first and second diffusion windows (18A, 18B) are diametrically opposed, preferably the first and second diffusion windows (18A, 18B) are aligned with first and second selector openings of the scent intensity selector for symmetrically covering or uncovering the first diffusion window (18A) and the second diffusion window (18B).

9. The air freshener (1) according to any one of the preceding claims, wherein the housing shroud (16) comprises a length, wherein the scent intensity selector (20) is laterally slidable along the length of the housing shroud (16) in a direction parallel to the center longitudinal axis (100).

10. The air freshener (1) according to any one of the preceding claims, wherein the housing shroud (16) comprises a circular shape, wherein the scent intensity selector (20) is rotatable about a center axis of the housing shroud (16).

11. The air freshener (1) according to claim 10 wherein the selector (20) is rotatable through a range of rotation angles up to 90 degrees, preferably from 1 degree to 85 degrees, more preferably from 5 degrees to 80 degrees, even more preferably from 10 degrees to 75 degrees; optionally, the scent intensity selector (20) further comprises a digitally engageable protrusion (25) to single handedly adjust the scent intensity selector (20).

12. The air freshener (1) according to any one of the preceding claims, wherein the perfume containing body (2) comprises a container (6) containing a volatile composition (8) and a membrane (9) in fluid communication with the volatile composition (8), wherein the exterior surface (90) of the membrane (9) forms at least a portion of said exterior evaporative surface (4) of the perfume containing body (2);
wherein an actuator (70) movable relative to the housing (10) is disposed adjacent to the membrane (9) such that moving the actuator (70) activates release of the volatile composition (8) from the perfume containing body (2).

13. The air freshener (1) according to any one of the preceding claims, wherein the housing (10) comprises a rear housing part (14) and a mounting portion (80) operably attachable to the rear housing part (14) for attaching the air freshener (1) to an interior surface in an interior space, preferably the mounting portion (80) comprises a resilient clip (90) disposed adjacent the housing (10) in a pre-mounting state in which the resilient clip (90) does not store energy and arranged to exert a force on the interior surface upon attaching the resilient member (90) of the mounting portion (80) to the interior surface in a mounting state; preferably the resilient clip (90) and the housing (10) form a unitary unit, more preferably, the resilient clip (90) comprise plastic.

14. A method of varying scent intensity in an interior space, the method comprising:

(i) providing an air freshener (1) according to any one of the preceding claims in an interior space; and
(ii) adjusting the scent intensity selector (20) relative to the center of the front housing part (12) for dispensing a volatile composition from the perfume containing body to the interior space, wherein the scent intensity selector (20) has a range of selector positions relative to the center of the front housing part (12); wherein a desired scent intensity setting is identifiable by a position of the scent intensity selector (20) along the range of selector positions, preferably the scent intensity selector (20) has at least three selector positions (60A, 60B, 60C), more preferably, the scent intensity selector (20) has five selector positions (60A, 60B, 60C, 60D, 60D).

15. The method according to claim 14, wherein providing the air freshener (1) in the interior space comprises sliding the air freshener (1) over an interior surface to attach the air freshener (1) to the interior surface, preferably the interior space is an automobile interior space, and the interior surface is selected from the group consisting of: automobile sun visor, automobile seat rear pocket, automobile ceiling grab handle, car door pocket, preferably, the interior surface is an automobile sun visor.

**Patentansprüche**

1. Lufterfrischer (1), der in der Lage ist, Duftintensität zu variieren, wobei der Lufterfrischer (1) betriebsfähig mit einem Befestigungsabschnitt verbindbar ist, um den Lufterfrischer (1) lösbar an einer Innenoberfläche in einem Innenbereich anzubringen, wobei der Lufterfrischer (1) Folgendes umfasst:

(a) ein Gehäuse (10) mit einem vorderseitigen Gehäuseteil (12) und einer Gehäusehülle (16), wobei die Gehäusehülle (16) mindestens einen Teil des vorderseitigen Gehäuseteils (12) abgrenzt, um eine Seite des Gehäuses zu definieren;
(b) eine Mittellängsachse (100), die sich entlang einer Länge des vorderseitigen Gehäuseteils (12) erstreckt und sich durch eine Mitte des vorderseitigen Gehäuseteils (12) erstreckt;
(c) ein Diffusionsfenster (18), das in der Gehäusehülle (16) angeordnet ist, wobei das Diffusionsfenster (18) eine Diffusionsfensterquerschnittsebene (19) aufweist, die sich über das Diffusionsfenster (18) erstreckt, wobei die Diffusionsfensterschnittebene (19) einen Diffusionsfensterflächenschwerpunkt (21) aufweist;
(d) einen duftstoffhaltigen Körper (2), der innerhalb des Gehäuses (10) in Fluidverbindung mit dem Diffusionsfenster (18) enthalten ist, wobei der duftstoffhaltige Körper (2) eine äußere Verdampfungsoberfläche (4) umfasst;
(e) wobei eine Luftstromachse (200), die den Diffusionsfensterflächenschwerpunkt (21) schneidet, einen Diffusionsweg (201) definiert, wobei der definierte Diffusionsweg (201) mindestens teilweise durch die äußere Verdampfungsoberfläche (4) des duftstoffhaltigen Körpers (2) gebildet ist, wobei eine diffusionswegbildende äußere Verdampfungsoberfläche (42) in Fluidverbindung mit dem definierten Diffusionsweg (201) steht und eine diffusionswegbildende äußere Verdampfungsoberflächen-Querschnittsfläche (42A) von 5 cm$^2$ bis 100 cm$^2$ aufweist;

(f) wobei der definierte Diffusionsweg (201) eine minimale Querschnittsfläche (201A) von mindestens 0,2 cm$^2$, gemessen entlang der Länge der Luftstromachse (200) und mindestens entlang der Länge (42D) der diffusionswegbildenden äußeren Verdampfungsoberfläche (42), umfasst; und

(g) einen Duftintensitätsselektor (20), der einstellbar an der Seite des Gehäuses (10) angebracht ist, um das Diffusionsfenster (18) mindestens teilweise zu bedecken oder freizulegen.

2. Lufterfrischer (1) nach Anspruch 1, wobei ein Verhältnis einer durchschnittlichen Dampffreisetzungsrate des Lufterfrischers (1) bei einer maximalen Duftintensitätseinstellung des Selektors (20) zu einer durchschnittlichen Dampffreisetzungsrate des Lufterfrischers (1) bei einer minimalen Duftintensitätseinstellung des Selektors (20) von etwa 1,3 bis etwa 75 beträgt.

3. Lufterfrischer (1) nach Anspruch 1 oder Anspruch 2, wobei die Gehäusehülle (16) Folgendes umfasst:

eine erste Hüllensektion (50A) und eine zweite Hüllensektion (50B), die der ersten Hüllensektion (50A) gegenüberliegt, wobei die erste und zweite Hüllensektion (50A, 50B) um mindestens einen Teil des vorderseitigen Gehäuseteils (12) herum angeordnet sind;
wobei die erste Hüllensektion (50A) ein erstes Diffusionsfenster (18A) umfasst, das eine erste Diffusionsfensterquerschnittsebene (19A) aufweist, die sich über das erste Diffusionsfenster (18A) erstreckt, wobei die erste Diffusionsfensterquerschnittsebene (19A) einen ersten Diffusionsflächenschwerpunkt (21A) aufweist;
wobei die zweite Hüllensektion (50B) ein zweites Diffusionsfenster (18B) umfasst, das eine zweite Diffusionsfensterquerschnittsebene (19B) aufweist, die sich über das zweite Diffusionsfenster (18B) erstreckt, wobei die zweite Diffusionsfensterquerschnittsebene (19B) einen zweiten Diffusionsfensterflächenschwerpunkt (21B) aufweist;
wobei die Luftstromachse (200) den ersten Diffusionsfensterflächenschwerpunkt (21A) und den zweiten Diffusionsfensterflächenschwerpunkt (21B) schneidet, wodurch ein Diffusionsweg (201) dazwischen definiert wird;
wobei der definierte Diffusionsweg (201) mindestens teilweise durch die äußere Verdampfungsoberfläche (4) des duftstoffhaltigen Körpers (2) gebildet ist, wobei die diffusionswegbildende äußere Verdampfungsoberfläche (42) in Fluidverbindung mit dem definierten Diffusionsweg (201) steht, wobei der Duftintensitätsselektor (20) einstellbar an der Gehäusehülle (16) angebracht ist, um das erste Diffusionsfenster (18A) und das zweite Diffusionsfenster (18B) mindestens teilweise zu bedecken oder freizulegen.

4. Lufterfrischer (1) nach Anspruch 3, wobei die diffusionswegbildende äußere Verdampfungsoberfläche (42) eine diffusionswegbildende äußere Verdampfungsoberflächen-Querschnittsfläche (42A) von 5 cm$^2$ bis 15 cm$^2$, vorzugsweise 5 cm$^2$ bis 10 cm$^2$, mehr bevorzugt 7 cm$^2$ bis 10 cm$^2$ aufweist;
wobei die erste Diffusionsfensterquerschnittsebene (19A) und die zweite Diffusionsfensterquerschnittsebene (19B) relativ zu der diffusionswegbildenden äußeren Verdampfungsoberflächen-Querschnittsfläche (42A) jeweils unabhängig eine Querschnittsfläche von mindestens 35 %, vorzugsweise 35 % bis 150 %, mehr bevorzugt 80 % bis 150 %, noch mehr bevorzugt 100 % bis 150 % aufweisen.

5. Lufterfrischer (1) nach Anspruch 4, wobei die minimale Querschnittsfläche (201A) des definierten Diffusionswegs (201) 0,2 cm$^2$ bis 10 cm$^2$, vorzugsweise 3 cm$^2$ bis 9 cm$^2$, mehr bevorzugt von 4 cm$^2$ bis 8 cm$^2$, noch mehr bevorzugt von 5 cm$^2$ bis 7 cm$^2$ beträgt.

6. Lufterfrischer (1) nach Anspruch 3, wobei die diffusionswegbildende äußere Verdampfungsoberfläche (42) eine diffusionswegbildende äußere Verdampfungsoberflächen-Querschnittsfläche (42A) von 20 cm$^2$ bis 50 cm$^2$, vorzugsweise 20 cm$^2$ bis 40 cm$^2$, mehr bevorzugt 25 cm$^2$ bis 30 cm$^2$ aufweist;
wobei die erste Diffusionsfensterquerschnittsebene (19A) und die zweite Diffusionsfensterquerschnittsebene (19B) relativ zu der diffusionswegbildenden äußeren Verdampfungsoberflächen-Querschnittsfläche (42A) jeweils unabhängig eine Querschnittsfläche von mindestens 0,9 %, vorzugsweise 0,9 % bis 100 %, mehr bevorzugt 1 % bis 80 %, noch mehr bevorzugt 10 % bis 70 %, sogar noch mehr bevorzugt 1 % bis 15 % aufweisen.

7. Lufterfrischer (1) nach Anspruch 6, wobei die minimale Querschnittsfläche (201A) des definierten Diffusionswegs (201) 0,2 cm$^2$ bis 10 cm$^2$, vorzugsweise 3 cm$^2$ bis 9 cm$^2$, mehr bevorzugt von 4 cm$^2$ bis 8 cm$^2$, noch mehr bevorzugt von 5 cm$^2$ bis 7 cm$^2$ beträgt.

8. Lufterfrischer (1) nach einem der Ansprüche 3 bis 7, wobei sich das erste und zweite Diffusionsfenster (18A, 18B) diametral gegenüberliegen, vorzugsweise das erste und zweite Diffusionsfenster (18A, 18B) mit einer ersten und zweiten Selektoröffnung des Duftintensitätsselektors ausgerichtet sind, um das erste Diffusionsfenster (18A) und das zweite Diffusionsfenster (18B) symmetrisch zu bedecken oder freizulegen.

9. Lufterfrischer (1) nach einem der vorstehenden Ansprüche, wobei die Gehäusehülle (16) eine Länge umfasst, wobei der Duftintensitätsselektor (20) seitlich entlang der Länge der Gehäusehülle (16) in einer Richtung parallel zu der Mittellängsachse (100) verschiebbar ist.

10. Lufterfrischer (1) nach einem der vorstehenden Ansprüche, wobei die Gehäusehülle (16) eine kreisförmige Form umfasst, wobei der Duftintensitätsselektor (20) um eine Mittelachse der Gehäusehülle (16) herum drehbar ist.

11. Lufterfrischer (1) nach Anspruch 10, wobei der Selektor (20) über einen Bereich von Drehwinkeln bis zu 90 Grad, vorzugsweise von 1 Grad bis 85 Grad, mehr bevorzugt von 5 Grad bis 80 Grad, noch mehr bevorzugt von 10 Grad bis 75 Grad drehbar ist; wobei der Duftintensitätsselektor (20) wahlweise ferner einen digital in Eingriff bringbaren Vorsprung (25) umfasst, um den Duftintensitätsselektor (20) einhändig einzustellen.

12. Lufterfrischer (1) nach einem der vorstehenden Ansprüche, wobei der duftstoffhaltige Körper (2) einen Behälter (6), der eine flüchtige Zusammensetzung (8) enthält, und eine Membran (9) in Fluidverbindung mit der flüchtigen Zusammensetzung (8) umfasst, wobei die Außenoberfläche (90) der Membran (9) mindestens einen Abschnitt der äußeren Verdampfungsoberfläche (4) des duftstoffhaltigen Körpers (2) bildet; wobei ein Betätigungselement (70), das relativ zu dem Gehäuse (10) beweglich ist, benachbart zu der Membran (9) angeordnet ist, sodass Bewegen des Betätigungselements (70) Freisetzung der flüchtigen Zusammensetzung (8) aus dem duftstoffhaltigen Körper (2) aktiviert.

13. Lufterfrischer (1) nach einem der vorstehenden Ansprüche, wobei das Gehäuse (10) einen hinteren Gehäuseteil (14) und einen Befestigungsabschnitt (80) umfasst, der betriebsfähig an dem hinteren Gehäuseteil (14) angebracht werden kann, um den Lufterfrischer (1) an einer Innenoberfläche in einem Innenbereich anzubringen, wobei der Befestigungsabschnitt (80) vorzugsweise eine elastische Klammer (90) umfasst, die in einem Vormontagezustand, in dem die elastische Klammer (90) keine Energie speichert, dem Gehäuse (10) angrenzend angeordnet ist und arrangiert ist, um beim Anbringen des elastischen Elements (90) des Befestigungsabschnitts (80) an der Innenoberfläche in einem Montagezustand eine Kraft auf die Innenoberfläche auszuüben; wobei die elastische Klammer (90) und das Gehäuse (10) vorzugsweise eine einheitliche Einheit bilden, wobei die elastische Klammer (90) mehr bevorzugt Kunststoff umfassen.

14. Verfahren zum Variieren von Duftintensität in einem Innenbereich, wobei das Verfahren Folgendes umfasst:

   (i) Bereitstellen eines Lufterfrischers (1) nach einem der vorstehenden Ansprüche in einem Innenbereich; und
   (ii) Einstellen des Duftintensitätsselektors (20) relativ zu der Mitte des vorderseitigen Gehäuseteils (12) zum Abgeben einer flüchtigen Zusammensetzung aus dem duftstoffhaltigen Körper in den Innenbereich, wobei der Duftintensitätsselektor (20) einen Bereich von Selektorpositionen relativ zu der Mitte des vorderseitigen Gehäuseteils (12) aufweist; wobei eine gewünschte Duftintensitätseinstellung durch eine Position des Duftintensitätsselektors (20) entlang des Bereichs von Selektorpositionen identifizierbar ist, wobei der Duftintensitätsselektor (20) vorzugsweise mindestens drei Selektorpositionen (60A, 60B, 60C) aufweist, wobei der Duftintensitätsselektor (20) mehr bevorzugt fünf Selektorpositionen (60A, 60B, 60C, 60D, 60D) aufweist.

15. Verfahren nach Anspruch 14, wobei das Bereitstellen des Lufterfrischers (1) in dem Innenbereich das Verschieben des Lufterfrischers (1) über eine Innenoberfläche umfasst, um den Lufterfrischer (1) an der Innenoberfläche anzubringen, wobei der Innenbereich vorzugsweise ein Automobilinnenbereich ist, und die Innenoberfläche ausgewählt ist aus der Gruppe bestehend aus: Automobilsonnenblende, Automobilsitzrückentasche, Automobildeckenhandgriff, Autotürentasche, wobei die Innenoberfläche vorzugsweise eine Automobilsonnenblende ist.

**Revendications**

1. Désodorisant d'ambiance (1) susceptible de faire varier l'intensité de parfum, le désodorisant d'ambiance (1) pouvant être raccordé de manière opérationnelle à une portion de montage pour fixer de manière amovible le désodorisant d'ambiance (1) à une surface intérieure dans un espace intérieur, le désodorisant d'ambiance (1) comprenant :

(a) un boîtier (10) ayant une partie de boîtier avant (12) et une enveloppe de boîtier (16), dans lequel l'enveloppe de boîtier (16) entoure au moins une partie de la partie de boîtier avant (12) pour définir un côté du boîtier ;

(b) un axe longitudinal central (100) s'étendant le long d'une longueur de la partie de boîtier avant (12) et s'étendant à travers un centre de la partie de boîtier avant (12) ;

(c) une fenêtre de diffusion (18) disposée dans l'enveloppe de boîtier (16), la fenêtre de diffusion (18) ayant un plan en coupe transversale de fenêtre de diffusion (19) s'étendant à travers la fenêtre de diffusion (18), dans lequel le plan en coupe de fenêtre de diffusion (19) a un centroïde de fenêtre de diffusion (21) ;

(d) un corps contenant du parfum (2) contenu dans le boîtier (10) en communication du point de vue des fluides avec la fenêtre de diffusion (18), dans lequel le corps contenant du parfum (2) comprend une surface d'évaporation extérieure (4) ;

(e) un axe de flux d'air (200) croisant le centroïde de fenêtre de diffusion (21) définissant une trajectoire de diffusion (201), dans lequel la trajectoire de diffusion définie (201) est au moins partiellement formée par la surface d'évaporation extérieure (4) du corps contenant du parfum (2), dans lequel une trajectoire de diffusion formant une surface d'évaporation extérieure (42) est en communication du point de vue des fluides avec la trajectoire de diffusion définie (201) et a une aire en coupe transversale de trajectoire de diffusion formant une surface d'évaporation extérieure (42A) de 5 cm$^2$ à 100 cm$^2$ ;

(f) dans lequel la trajectoire de diffusion définie (201) comprend une aire en coupe transversale minimum (201A) d'au moins 0,2 cm$^2$ mesurée le long de la longueur de l'axe de flux d'air (200) et au moins le long de la longueur (42D) de la trajectoire de diffusion formant une surface d'évaporation extérieure (42) ; et

(g) un sélecteur d'intensité de parfum (20) fixé de manière réglable au côté du boîtier (10) pour recouvrir ou découvrir au moins partiellement la fenêtre de diffusion (18).

2. Désodorisant d'ambiance (1) selon la revendication 1, dans lequel un rapport d'une vitesse moyenne de libération de vapeur du désodorisant d'ambiance (1) à un paramètre d'intensité de parfum maximum du sélecteur (20) à une vitesse moyenne de libération de vapeur du désodorisant d'ambiance (1) à un paramètre d'intensité de parfum minimum du sélecteur (20) est d'environ 1,3 à environ 75.

3. Désodorisant d'ambiance (1) selon la revendication 1 ou la revendication 2, dans lequel l'enveloppe de boîtier (16) comprend :

une première section d'enveloppe (50A) et une deuxième section d'enveloppe (50B) opposée à la première section d'enveloppe (50A), dans lequel les première et deuxième sections d'enveloppe (50A, 50B) sont disposées autour d'au moins une partie de la partie de boîtier avant (12) ;

dans lequel la première section d'enveloppe (50A) comprend une première fenêtre de diffusion (18A) ayant un premier plan en coupe transversale de fenêtre de diffusion (19A) s'étendant à travers la première fenêtre de diffusion (18A), dans lequel le premier plan en coupe de fenêtre de diffusion (19A) a un premier centroïde de diffusion (21A) ;

dans lequel la deuxième section d'enveloppe (50B) comprend une deuxième fenêtre de diffusion (18B) ayant un deuxième plan en coupe transversale de fenêtre de diffusion (19B) s'étendant à travers la deuxième fenêtre de diffusion (18B), dans lequel le deuxième plan en coupe transversale de fenêtre de diffusion (19B) a un deuxième centroïde de fenêtre de diffusion (21B) ;

dans lequel l'axe de flux d'air (200) croise le premier centroïde de fenêtre de diffusion (21A) et le deuxième centroïde de fenêtre de diffusion (21B) définissant une trajectoire de diffusion (201) entre ceux-ci ; dans lequel la trajectoire de diffusion définie (201) est au moins partiellement formée par la surface d'évaporation extérieure (4) du corps contenant du parfum (2), dans lequel la trajectoire de diffusion formant une surface d'évaporation extérieure (42) est en communication du point de vue des fluides avec la trajectoire de diffusion définie (201) dans lequel le sélecteur d'intensité de parfum (20) est fixé de manière réglable à l'enveloppe de boîtier (16) pour recouvrir ou découvrir au moins partiellement la première fenêtre de diffusion (18A) et la deuxième fenêtre de diffusion (18B).

4. Désodorisant d'ambiance (1) selon la revendication 3, dans lequel la trajectoire de diffusion formant une surface d'évaporation extérieure (42) a une aire en coupe transversale de trajectoire de diffusion formant une surface d'évaporation extérieure (42A) de 5 cm$^2$ à 15 cm$^2$, de préférence de 5 cm$^2$ à 10 cm$^2$, plus préférablement de 7 cm$^2$ à 10 cm$^2$ ;

dans lequel le premier plan en coupe transversale de fenêtre de diffusion (19A) et le deuxième plan en coupe transversale de fenêtre de diffusion (19B) ont chacun indépendamment une aire en coupe transversale d'au moins 35 %, de préférence de 35 % à 150 %, plus préférablement de 80 % à 150 %, encore plus préférablement de 100 % à 150 % relativement à l'aire en coupe transversale de trajectoire de diffusion formant une surface d'évaporation extérieure (42A).

5. Désodorisant d'ambiance (1) selon la revendication 4, dans lequel l'aire en coupe transversale minimum (201A) de la trajectoire de diffusion définie (201) est de 0,2 cm$^2$ à 10 cm$^2$, de préférence de 3 cm$^2$ à 9 cm$^2$, plus préférablement de 4 cm$^2$ à 8 cm$^2$, encore plus préférablement de 5 cm$^2$ à 7 cm$^2$.

6. Désodorisant d'ambiance (1) selon la revendication 3, dans lequel la trajectoire de diffusion formant une surface d'évaporation extérieure (42) a une aire en coupe transversale de trajectoire de diffusion formant une surface d'évaporation extérieure (42A) de 20 cm$^2$ à 50 cm$^2$, de préférence de 20 cm$^2$ à 40 cm$^2$, plus préférablement de 25 cm$^2$ à 30 cm$^2$ ;
dans lequel le premier plan en coupe transversale de fenêtre de diffusion (19A) et le deuxième plan en coupe transversale de fenêtre de diffusion (19B) ont chacun indépendamment une aire en coupe transversale d'au moins 0,9 %, de préférence de 0,9 % à 100 %, plus préférablement de 1 % à 80 %, encore plus préférablement de 10 % à 70 %, et encore plus préférablement de 1 % à 15 % relativement à l'aire en coupe transversale de trajectoire de diffusion formant une surface d'évaporation extérieure (42A).

7. Désodorisant d'ambiance (1) selon la revendication 6, dans lequel l'aire en coupe transversale minimum (201A) de la trajectoire de diffusion définie (201) est de 0,2 cm$^2$ à 10 cm$^2$, de préférence de 3 cm$^2$ à 9 cm$^2$, plus préférablement de 4 cm$^2$ à 8 cm$^2$, encore plus préférablement de 5 cm$^2$ à 7 cm$^2$.

8. Désodorisant d'ambiance (1) selon l'une quelconque des revendications 3 à 7, dans lequel les première et deuxième fenêtres de diffusion (18A, 18B) sont diamétralement opposées, de préférence les première et deuxième fenêtres de diffusion (18A, 18B) sont alignées avec des première et deuxième ouvertures de sélecteur du sélecteur d'intensité de parfum pour recouvrir ou découvrir symétriquement la première fenêtre de diffusion (18A) et la deuxième fenêtre de diffusion (18B).

9. Désodorisant d'ambiance (1) selon l'une quelconque des revendications précédentes, dans lequel l'enveloppe de boîtier (16) comprend une longueur, dans lequel le sélecteur d'intensité de parfum (20) est coulissant latéralement le long de la longueur de l'enveloppe de boîtier (16) dans une direction parallèle à l'axe longitudinal central (100).

10. Désodorisant d'ambiance (1) selon l'une quelconque des revendications précédentes, dans lequel l'enveloppe de boîtier (16) comprend une forme circulaire, dans lequel le sélecteur d'intensité de parfum (20) est rotatif autour d'un axe central de l'enveloppe de boîtier (16).

11. Désodorisant d'ambiance (1) selon la revendication 10 dans lequel le sélecteur (20) est rotatif à travers une plage d'angles de rotation jusqu'à 90 degrés, de préférence de 1 degré à 85 degrés, plus préférablement de 5 degrés à 80 degrés, encore plus préférablement de 10 degrés à 75 degrés ; éventuellement, le sélecteur d'intensité de parfum (20) comprend en outre une saillie pouvant venir en prise de manière numérique (25) pour régler d'une seule main le sélecteur d'intensité de parfum (20).

12. Désodorisant d'ambiance (1) selon l'une quelconque des revendications précédentes, dans lequel le corps contenant du parfum (2) comprend un récipient (6) contenant une composition volatile (8) et une membrane (9) en communication du point de vue des fluides avec la composition volatile (8), dans lequel la surface extérieure (90) de la membrane (9) forme au moins une portion de ladite surface d'évaporation extérieure (4) du corps contenant du parfum (2) ;
dans lequel un actionneur (70) mobile relativement au boîtier (10) est disposé adjacent à la membrane (9) de telle sorte que le déplacement de l'actionneur (70) active la libération de la composition volatile (8) du corps contenant du parfum (2).

13. Désodorisant d'ambiance (1) selon l'une quelconque des revendications précédentes, dans lequel le boîtier (10) comprend une partie de boîtier arrière (14) et une portion de montage (80) pouvant être fixée de manière opérationnelle à la partie de boîtier arrière (14) pour fixer le désodorisant d'ambiance (1) à une surface intérieure dans un espace intérieur, de préférence la portion de montage (80) comprend une attache élastique (90) disposée adjacente au boîtier (10) dans un état de prémontage où l'attache élastique (90) ne stocke pas d'énergie et disposée pour exercer une force sur la surface intérieure après avoir fixé le membre élastique (90) de la portion de montage (80) à la surface intérieure dans un état de montage ; de préférence l'attache élastique (90) et le boîtier (10) forment un motif d'un seul tenant, plus préférablement, l'attache élastique (90) comprend du plastique.

**14.** Procédé de variation de l'intensité de parfum dans un espace intérieur, le procédé comprenant :

(i) la fourniture d'un désodorisant d'ambiance (1) selon l'une quelconque des revendications précédentes dans un espace intérieur ; et

(ii) le réglage du sélecteur d'intensité de parfum (20) relativement au centre de la partie de boîtier avant (12) pour distribuer une composition volatile du corps contenant du parfum à l'espace intérieur, dans lequel le sélecteur d'intensité de parfum (20) a une plage de positions de sélecteur relativement au centre de la partie de boîtier avant (12) ; dans lequel un paramètre d'intensité de parfum souhaité est identifiable par une position du sélecteur d'intensité de parfum (20) le long de la plage de positions de sélecteur, de préférence le sélecteur d'intensité de parfum (20) a au moins trois positions de sélecteur (60A, 60B, 60C), plus préférablement, le sélecteur d'intensité de parfum (20) a cinq positions de sélecteur (60A, 60B, 60C, 60D, 60D).

**15.** Procédé selon la revendication 14, dans lequel la fourniture du désodorisant d'ambiance (1) dans l'espace intérieur comprend le coulissement du désodorisant d'ambiance (1) sur une surface intérieure pour fixer le désodorisant d'ambiance (1) à la surface intérieure, de préférence l'espace intérieur est un espace intérieur d'automobile, et la surface intérieure est choisie dans le groupe constitué de : pare-soleil d'automobile, poche arrière de siège d'automobile, poignée de maintien de plafond d'automobile, poche de porte de voiture, de préférence, la surface intérieure est un pare-soleil d'automobile.

EP 3 682 910 B1

FIG. 1

29

FIG. 2A

FIG. 2B

FIG. 3A

FIG. 3B

FIG. 3C

FIG. 4A

FIG. 4B

FIG. 5

FIG. 6A

FIG. 6B

FIG. 7A

FIG. 7B

FIG. 8A          FIG. 8B

FIG. 9A          FIG. 9B

FIG. 10A          FIG. 10B

EP 3 682 910 B1

FIG. 11A

FIG. 11B

FIG. 12

FIG. 13

FIG. 14

FIG. 15

FIG. 16

FIG. 17

FIG. 18

FIG. 19

FIG. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5478505 A
- US 2010308126 A1
- US 4666638 A